(19) Europäisches Patentamt / European Patent Office / Office européen des brevets

(11) **EP 4 048 249 B1**

(12) **EUROPEAN PATENT SPECIFICATION**

(45) Date of publication and mention
of the grant of the patent:
**03.09.2025   Bulletin 2025/36**

(21) Application number: **20879266.3**

(22) Date of filing: **23.10.2020**

(51) International Patent Classification (IPC):
*A61K 31/422* (2006.01)     *A61P 25/00* (2006.01)
*A61K 31/18* (2006.01)     *A61K 31/404* (2006.01)
*A61K 31/4184* (2006.01)     *A61K 31/498* (2006.01)
*A61K 31/17* (2006.01)     *A61K 31/4045* (2006.01)
*A61K 31/4196* (2006.01)     *A61K 31/403* (2006.01)
*A61K 31/454* (2006.01)     *A61K 31/496* (2006.01)
*A61K 31/506* (2006.01)

(52) Cooperative Patent Classification (CPC):
A61K 31/506; A61K 31/17; A61K 31/403;
A61K 31/404; A61K 31/4045; A61K 31/4196;
A61K 31/422; A61K 31/454; A61K 31/496;
A61P 25/00

(86) International application number:
**PCT/US2020/057133**

(87) International publication number:
**WO 2021/081376 (29.04.2021 Gazette 2021/17)**

(54) **METHODS OF TREATING THE SYMPTOMS OF AUTISM SPECTRUM DISORDER**

VERFAHREN ZUR BEHANDLUNG DER SYMPTOME VON AUTISMUS-SPEKTRUMSSTÖRUNGEN

MÉTHODES DE TRAITEMENT DES SYMPTÔMES DU TROUBLE DU SPECTRE AUTISTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **23.10.2019   US 201962925023 P**
**17.04.2020   US 202063011715 P**

(43) Date of publication of application:
**31.08.2022   Bulletin 2022/35**

(73) Proprietor: **MapLight Therapeutics, Inc.**
**Redwood City, CA 94063 (US)**

(72) Inventors:
• **WOOD, Michael**
**San Francisco, California 94107 (US)**
• **LILLIE, James**
**San Francisco, California 94107 (US)**

(74) Representative: **Cooley (UK) LLP**
**22 Bishopsgate**
**London EC2N 4BQ (GB)**

(56) References cited:
US-A1- 2017 081 273     US-A1- 2018 028 499
US-A1- 2018 028 499     US-B2- 8 716 300

• **WALSH JESSICA J ET AL: "5-HT release in nucleus accumbens rescues social deficits in mouse autism model", CLEO: APPLICATIONS AND TECHNOLOGY 2019 SAN JOSE, CALIFORNIA UNITED STATES 5-10 MAY 2019, OPTICA, vol. 560, no. 7720, 8 August 2018 (2018-08-08), pages 589 - 594, XP036579411, DOI: 10.1038/S41586-018-0416-4**

- LACIVITA ENZA ET AL: "Targets for Drug Therapy for Autism Spectrum Disorder: Challenges and Future Directions", JOURNAL OF MEDICINAL CHEMISTRY, vol. 60, no. 22, 22 November 2017 (2017-11-22), US, pages 9114 - 9141, XP055882290, ISSN: 0022-2623, Retrieved from the Internet <URL:https://pubs.acs.org/doi/pdf/10.1021/acs.jmedchem.7b00965> DOI: 10.1021/acs.jmedchem.7b00965
- GHANIZADEH AHMAD ET AL: "A Randomized Double-Blind Placebo-Controlled Clinical Trial of Adjuvant Buspirone for Irritability in Autism", PEDIATRIC NEUROLOGY, vol. 52, no. 1, 1 January 2015 (2015-01-01), NL, pages 77 - 81, XP093095879, ISSN: 0887-8994, DOI: 10.1016/j.pediatrneurol.2014.09.017
- DELEU DIRK ET AL: "Current and Emerging Second-Generation Triptans in Acute Migraine Therapy: A Comparative Review", THE JOURNAL OF CLINICAL PHARMACOLOGY, vol. 40, no. 7, 1 July 2000 (2000-07-01), US, pages 687 - 700, XP093096468, ISSN: 0091-2700, DOI: 10.1177/00912700022009431
- HOWES OLIVER D ET AL: "Autism spectrum disorder: Consensus guidelines on assessment, treatment and research from the British Association for Psychopharmacology", JOURNAL OF PSYCHOPHARMACOLOGY., vol. 32, no. 1, 1 January 2018 (2018-01-01), GB, pages 3 - 29, XP093096494, ISSN: 0269-8811, DOI: 10.1177/0269881117741766
- HOLLANDER ERIC ET AL: "The relationship between repetitive behaviors and growth hormone response to sumatriptan challenge in adult autistic disorder", NEUROPSYCHOPHARMACOLOGY, vol. 22, no. 2, 1 February 2000 (2000-02-01), pages 1 - 5, XP093095109
- SIEGEL ET AL.: "The autism inpatient collection: methods and preliminary sample description", MOLECULAR AUTISM, vol. 6, no. 61, 10 November 2015 (2015-11-10), pages 1 - 10, XP055819379

**Description**

**BACKGROUND**

Autism spectrum disorder (ASD) is a serious neurodevelopmental disorder characterized by stereotypic behaviors and deficits in language and social interaction. The reported incidence of autism has rapidly increased to 1 in 59 births in the United States as of 2014 (https://www.cdc.gov/mmwr/volumes/67/ss/ss6706a1.htm), representing a significant medical and social burden in the coming decades. Consensus guidelines for the treatment of autism spectrum disorder have been published for example by the British Association for Psychopharmacology (Journal of psychopharmacology. 2000; 22(2):1-5). To date, there are no FDA-approved treatments for reducing or eliminating the core symptoms of autism spectrum disorder. There is a need in the art for methods for treating and reducing the severity and incidence of symptoms associated with autism spectrum disorder, including increased irritability and decreased sociability.

**SUMMARY**

In a first aspect of the invention, there is provided a therapeutically effective amount of zolmitriptan or a pharmaceutically acceptable salt thereof for use in decreasing irritability or improving sociability associated with autism spectrum disorder (ASD).

Preferred embodiments of the invention in any of its various aspects are as described below or as defined in the sub claims.

All aspects, embodiments and disclosures described below are not part of the present invention unless they are encompassed by the scope of the appended claims.

In addition, references to methods of treatment by therapy, surgery or in vivo diagnosis are to be interpreted as references to compounds, pharmaceutic compositions and medicaments for use in those methods.

The present disclosure provides for reference methods of treating the symptoms associated with autism spectrum disorder (ASD) comprising administering a therapeutically effective amount of a triptan to a patient in need thereof.

The present disclosure provides for reference methods of treating the symptoms associated with ASD comprising administering a therapeutically effective amount of zolmitriptan or a pharmaceutically acceptable salt thereof to a patient in need thereof.

The present disclosure provides for reference methods of treating the symptoms associated with ASD comprising administering a therapeutically effective amount of sumatriptan or a pharmaceutically acceptable salt thereof to a patient in need thereof.

The present disclosure provides for reference methods of treating the symptoms associated with ASD comprising administering a therapeutically effective amount of a triptan to a patient in need thereof, wherein after said treating the patient experiences a substantial improvement in sociability compared to prior to said treating.

The present disclosure provides for reference a method of treating the symptoms associated with ASD comprising administering a therapeutically effective amount of a triptan to a patient in need thereof, wherein after said treating the patient experiences a substantial decrease in irritability associated with ASD compared to prior to said treating. The present disclosure provides for reference a method of treating aggression associated with autism spectrum disorder (ASD) comprising administering a therapeutically effective amount of a triptan to a patient in need thereof, wherein after said treating the patient experiences a substantial decrease in aggression associated with ASD compared to prior to said treating.

**BRIEF DESCRIPTION OF THE DRAWINGS**

**FIG. 1** shows the frequency of attacks in BALB/c resident mice cages from a cross-over Resident-Intruder (RI) study in which resident BALB/c mice were treated with vehicle control followed by 3 or 10 mg/kg zolmitriptan.

**FIG. 2** shows the attack time (secs) in BALB/c resident mice cages from a cross-over Resident-Intruder (RI) study in which resident BALB/c mice were treated with vehicle control followed by 3 or 10 mg/kg zolmitriptan.

**FIG. 3** shows the attack time (secs) of adult male CD-1 mice from a cross-over Resident-Intruder (RI) study in which resident CD-1 mice were treated with vehicle control, 10 mg/kg zolmitriptan or 0.03 mg/kg risperidone. Attack time was measured over a 5 min period as a cross over design. Data are expressed as mean $\pm$ standard error of means.

**FIG. 4** shows the sociability index in a valproic acid (VPA)-induced c57/Bl6 mouse model of Autism Spectrum Disorder in mice treated with vehicle control or 10 mg/kg zolmitriptan.

**FIG. 5** shows the average CSF levels of zolmitriptan and its metabolite N-desmethyl zolmitriptan (NDMZ) following oral administration of 5 mg, 10 mg, 20 mg, and 30 mg doses to human subjects.

**DEFINITIONS**

For convenience, certain terms employed in the specification, examples and claims are collected here. Unless defined otherwise, all technical and scientific terms used in this disclosure have the same meanings as commonly understood by one of ordinary skill in the art to which this disclosure belongs.

The term "about" when immediately preceding a numerical value means a range (e.g., plus or minus 10% of that value). For example, "about 50" can mean 45 to 55, "about 25,000" can mean 22,500 to 27,500, etc., unless the context of the disclosure indicates otherwise, or is inconsistent with such an interpretation. For example in a list of numerical values such as "about 49, about 50, about 55, ...", "about 50" means a range extending to less than half the interval(s) between the preceding and subsequent values, e.g., more than 49.5 to less than 52.5. Furthermore, the phrases "less than about" a value or "greater than about" a value should be understood in view of the definition of the term "about" provided herein. Similarly, the term "about" when preceding a series of numerical values or a range of values (e.g., "about 10, 20, 30" or "about 10-30") refers, respectively to all values in the series, or the endpoints of the range.

The terms "administer," "administering" or "administration" as used herein refer to either directly administering a compound or pharmaceutically acceptable salt or ester of the compound or a composition comprising the compound or pharmaceutically acceptable salt or ester of the compound to a patient.

The terms "effective amount" and "therapeutically effective amount" are used interchangeably in this disclosure and refer to an amount of a compound, or a salt, solvate or ester thereof, that, when administered to a patient, is capable of performing the intended result. For example, an effective amount of a triptan is that amount that is required to reduce at least one symptom of ASD in a patient, e.g. the amount required to improve irritability associated with ASD, reduce aggression associated with ASD, or increase sociability in a patient. The actual amount that comprises the "effective amount" or "therapeutically effective amount" will vary depending on a number of conditions including, but not limited to, the triptan that is administered, the severity of the disorder, the size and health of the patient, and the route of administration. A skilled medical practitioner can readily determine the appropriate amount using the methods described herein and as known in the medical arts.

The phrase "pharmaceutically acceptable" as used herein refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The term "salts" as used herein embraces pharmaceutically acceptable salts commonly used to form alkali metal salts of free acids and to form addition salts of free bases. The nature of the salt is not critical, provided that it is pharmaceutically acceptable. The term "salts" also includes solvates of addition salts, such as hydrates, as well as polymorphs of addition salts. Suitable pharmaceutically acceptable acid addition salts can be prepared from an inorganic acid or from an organic acid. Examples of such inorganic acids are hydrochloric, hydrobromic, hydroiodic, nitric, carbonic, sulfuric, and phosphoric acid. Appropriate organic acids can be selected from aliphatic, cycloaliphatic, aromatic, arylaliphatic, and heterocyclyl containing carboxylic acids and sulfonic acids, for example formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, stearic, salicylic, p-hydroxybenzoic, phenylacetic, mandelic, embonic (pamoic), methanesulfonic, ethanesulfonic, benzenesulfonic, pantothenic, toluenesulfonic, 2-hydroxyethanesulfonic, sulfanilic, cyclohexylaminosulfonic, algenic, 3-hydroxybutyric, galactaric and galacturonic acid.

The term "treating" as used herein with regard to a patient, refers to improving at least one symptom of the patient's disorder. Treating can be improving or at least partially ameliorating a disorder. For example, a patient's autism spectrum disorder is treated when the method reduces at least one symptom of ASD, e.g., irritability, aggression, or decreased sociability, in the patient.

The term "therapeutic effect" as used herein refers to a desired or beneficial effect provided by the method and/or the composition. For example, the method for treating autism spectrum disorder provides a therapeutic effect when the method improves at least one symptom of ASD, e.g., an improvement in irritability associated with ASD, an improvement in aggression associated with ASD, or increased sociability, in a patient.

**DETAILED DESCRIPTION OF THE INVENTION**

Autism spectrum disorder (ASD) is a neurodevelopmental disorder that is characterized by impairments in social interaction and communication, restricted interests, and repetitive behavior. Individuals on the autism spectrum experience widely varying degrees and types of impairments, from mild to severe. Although early detection and interventions are encouraged to maximize the benefits and reduce the severity of the symptoms, individuals of any age can benefit from interventions and therapies that can reduce symptoms and increase skills and abilities. Appropriate subjects for the methods described herein include, without limitation, humans diagnosed as having or suspected of having autism spectrum disorder.

The present disclosure provides for reference methods of treating one or more symptoms of ASD by administering a therapeutically effective amount of a triptan to a patient in need thereof.

The present disclosure provides for reference methods of treating the symptoms of a condition selected from Asperger's disorder or pervasive developmental disorder not otherwise specified (PDD-NOS) by administering a therapeutically effective amount of a triptan to a patient in need thereof.

The present disclosure provides for reference methods of improving the one or more symptoms associated with ASD by administering a therapeutically effective amount of a triptan to a patient in need thereof. In some embodiments, the methods of the present disclosure provide an improvement in sociability compared to prior to the treatment. In some embodiments, the methods of the present disclosure provide a reduction in irritability associated with ASD compared to prior to the treatment. The methods of the present disclosure provide for reference a reduction in aggression associated with ASD compared to prior to the treatment.

**Triptans**

A triptan as employed in the present methods can form a part of a pharmaceutical composition by combining a triptan, or a pharmaceutically acceptable salt thereof, with a pharmaceutically acceptable carrier. Additionally, the compositions can include an additive selected from the group consisting of adjuvants, excipients, diluents, release-modifying agents and stabilizers. The composition can be an immediate release formulation, a delayed release formulation, a sustained release formulation or an extended release formulation.

Triptans belong to the serotonin receptor subtype-selective drug class. Triptans have selective activity on the 5-$HT_{1B}$, 5-$HT_{1D}$, and 5-$HT_{1F}$ receptors, which belong to the serotonin (5-HT) system. Triptans exhibit vasoconstrictive properties, which are mediated by an action on 5-$HT_{1B}$ in arterial smooth muscle. Vasoconstriction by triptans leads to a dose-dependent increase in blood flow velocity in middle cerebral vessels. It is thought that triptans inhibit the abnormal activation of peripheral nociceptors. Triptans are presumed to reduce plasma protein extravasation (PPE) by inhibiting the activation of nociceptors and preventing the peripheral release of vasoactive peptides, including substance P and calcitonin gene related peptide (CGRP). Triptan binding sites also exist within the central nervous system. Thus, it is possible that triptans exhibit an effect on the central nervous system. At the time of this disclosure, triptans are FDA approved for the treatment of migraine headaches.

The serotonin system has been implicated in the pathophysiology of autism since an abnormal blood 5-HT level was discovered as the first biomarker of the disorder more than 50 years ago (Schain et al. J Pediatr. 1961 Mar;58:315-20). A major source of 5-HT in the brain originates from the midbrain dorsal raphe nucleus (DRN) that projects broadly across the cortex, amygdala, hypothalamus, and to other subcortical structures associated with emotional processing and social behavior. Dense serotonergic projections from the DRN innervate the nucleus accumbens (NAc), a well-conserved basal forebrain structure that acts as the integrator of motivational signals preceding the selection of behavioral actions (Kravitz et al. Physiology (Bethesda). 2012 Jun;27(3):167-77.). In the context of social interaction, NAc reward processing is thought to govern the choice of social approach vs. social avoidance behaviors (Pfaff. Trends Neurosci. 2019 Jul;42(7):448-457. ). Evidence from preclinical studies supports a critical role for 5-HT signaling in social behaviors and social reward (Kane et al. PLoS One. 2012;7(11):e48975.; Challis et al. J Neurosci. 2013 Aug 28;33(35):13978-88, 13988a.; Li et al. Nat Commun. 2016 Jan 28;7:10503; Walsh et al. Nature. 2018; 560(772): 589-594 .). Furthermore, aberrant processing of rewards in the NAc has been observed in fMRI imaging studies of children with ASD (Clements et al. JAMA Psychiatry. 2018 Aug 1;75(8):797-808. doi: 10.1001/jamapsychiatry.2018.1100.). An enhancement of risperidone effectiveness in the treatment of irritability in individuals with autism by the combination of buspirone, a 5HT agonist, has also been shown (Ghanizaden Ahmad et al. Pediatric neurology. 2015; 52(1): 77-81).

Within the human brain, 5-HT1B is highly expressed in the NAc (Garcia-Alloza et al. Neuropsychologia. 2005;43(3):442-9.; Varnas et al. Hum Brain Mapp. 2004 Jul;22(3):246-60.; Varnas et al. Synapse. 56: 21-8.). It is an inhibitory G protein coupled receptor localized in axon terminals that functions to suppress neurotransmitter release (Sari. Neurosci Biobehav Rev. 2004 Oct;28(6):565-82. ). As an autoreceptor, 5-HT1B inhibits the release of 5-HT, but it also plays an important role as a heteroreceptor to inhibit the release of other neurotransmitters including glutamate, GABA, dopamine and acetylcholine. (Boscher et al. (1994) Neuroscience 58:167-182.). According to the present disclosure, the 5-HT1B agonism of the triptans described herein modulates the behavior symptoms of ASD.

The triptan used in the formulations and methods of the present disclosure is zolmitriptan or a pharmaceutically acceptable salt thereof.

The methods of the present disclosure comprise administering a therapeutically effective amount of zolmitriptan or a pharmaceutically acceptable salt thereof to a patient in need.

**Formulations**

The methods of the present disclosure can employ various pharmaceutical compositions for administration to patients, e.g., humans and animals in unit dosage forms, such as tablets, capsules, pills, powders, granules, sterile parenteral solutions or suspensions, inhalable dry powders, dispersions, solutions or suspensions, and oral solutions or suspensions, and oil-water emulsions containing suitable quantities of a triptan, or pharmaceutically acceptable salts thereof.

A pharmaceutical composition of the present disclosure may be prepared for oral administration. A pharmaceutical composition may be formulated by combining one or more triptans and pharmaceutically acceptable carriers. Certain of such carriers enable pharmaceutical compositions to be formulated as tablets, pills, dragees, capsules, liquids, gels, syrups, slurries, suspensions and the like, for oral ingestion by a subject. Suitable excipients include, but are not limited to, fillers, such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). Such a mixture may be optionally ground and auxiliaries are optionally added. Pharmaceutical compositions may be formed to obtain tablets or dragee cores. Disintegrating agents (e.g., cross-linked polyvinyl pyrrolidone, agar, or alginic acid or a salt thereof, such as sodium alginate) may be added.

Aqueous carriers suitable for use in the present disclosure include, but are not limited to, water, ethanol, alcoholic/aqueous solutions, glycerol, emulsions or suspensions, including saline and buffered media. Oral carriers can be elixirs, syrups, capsules, tablets and the like.

Liquid carriers suitable for use in the present disclosure can be used in preparing solutions, suspensions, emulsions, syrups, and elixirs. The triptan can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators.

Liquid carriers suitable for use in the present application include, but are not limited to, water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil).

A pharmaceutical composition of the present disclosure may be prepared for administration by injection (e.g., intravenous, subcutaneous, intramuscular, etc.). A pharmaceutical composition may comprise a carrier and may be formulated in aqueous solution, such as water or physiologically compatible buffers such as Hanks's solution, Ringer's solution, or physiological saline buffer. Other ingredients may be included (e.g., ingredients that aid in solubility or serve as preservatives). Injectable suspensions may be prepared using appropriate liquid carriers, suspending agents and the like. Certain pharmaceutical compositions for injection are presented in unit dosage form, e.g., in ampoules or in multi-dose containers. Certain pharmaceutical compositions for injection are suspensions, solutions or emulsions in oily or aqueous vehicles, and may contain formulatory agents such as suspending, stabilizing and/or dispersing agents. Certain solvents suitable for use in pharmaceutical compositions for injection include, but are not limited to, lipophilic solvents and fatty oils, such as sesame oil, synthetic fatty acid esters, such as ethyl oleate or triglycerides, and liposomes. Aqueous injection suspensions may contain substances that increase the viscosity of the suspension, such as sodium carboxymethyl cellulose, sorbitol, or dextran. Optionally, such suspensions may also contain suitable stabilizers or agents that increase the solubility of the pharmaceutical agents to allow for the preparation of highly concentrated solutions.

The sterile injectable preparation may also be a sterile injectable solution or suspension in a non-toxic parenterally acceptable diluent or solvent, such as a solution in 1, 3-butane-diol or prepared as a lyophilized powder. Among the acceptable vehicles and solvents that may be employed are water, Ringer's solution and isotonic sodium chloride solution. In addition, sterile fixed oils may conventionally be employed as a solvent or suspending medium. For this purpose any bland fixed oil may be employed including synthetic mono- or diglycerides. In addition, fatty acids such as oleic acid may likewise be used in the preparation of injectables. Formulations for intravenous administration can comprise solutions in sterile isotonic aqueous buffer. Where necessary, the formulations can also include a solubilizing agent and a local anesthetic to ease pain at the site of the injection. Generally, the ingredients are supplied either separately or mixed together in unit dosage form, for example, as a dry lyophilized powder or water free concentrate in a hermetically sealed container such as an ampule or sachet indicating the quantity of active agent. Where the compound is to be administered by infusion, it can be dispensed in a formulation with an infusion bottle containing sterile pharmaceutical grade water, saline or dextrose/water. Where the compound is administered by injection, an ampule of sterile water for injection or saline can be provided so that the ingredients can be mixed prior to administration.

Suitable formulations further include aqueous and non-aqueous sterile injection solutions that can contain antioxidants, buffers, bacteriostats, bactericidal antibiotics and solutes that render the formulation isotonic with the bodily fluids of the intended recipient; and aqueous and non-aqueous sterile suspensions, which can include suspending agents and thickening agents.

A pharmaceutical composition of the present disclosure may be formulated as a depot preparation. Certain such depot preparations are typically longer acting than non-depot preparations. Such preparations may be administered by implantation (for example subcutaneously or intramuscularly) or by intramuscular injection. Depot preparations may be prepared using suitable polymeric or hydrophobic materials (for example an emulsion in an acceptable oil) or ion exchange resins, or as sparingly soluble derivatives, for example, as a sparingly soluble salt.

A pharmaceutical composition of the present disclosure may be prepared for intranasal administration.

## Administration and Dosing

The disclosure provides methods for treating the symptoms associated with autism spectrum disorder (ASD) by administering an effective amount of a triptan or a pharmaceutically acceptable salt thereof, to a patient in need thereof. An effective amount may be an amount sufficient to eliminate or significantly reduce one or more symptoms associated with ASD or to alleviate those symptoms (e.g., reduce the symptoms, such as irritability, aggression, or improve sociability, compared to the symptoms present prior to treatment). In some embodiments, an effective amount is an amount sufficient to eliminate or significantly reduce irritability associated with ASD as compared to prior to treatment. In some embodiments, an effective amount is an amount sufficient to eliminate or significantly improve sociability as compared to prior to treatment.

Reduction of the symptoms of autism in patients with ASD can be determined by various methods. The effectiveness of a dosage regimen can be determined by evaluation according to the OSU Autism Rating Scale (OARS-5), Vineland Adaptive Behavior Scales, ADOS-2, CGI-S and CGI-C social deficit subscales, autism behavior inventory, and the childhood autism rating scale, social responsiveness scale, aberrant behavior checklist, social developmental disorder behavior inventory, Top 3 Caregiver Concerns, the Autism Behavior Inventory, and the Autism Treatment Evaluation Checklist (ATEC), Social Responsiveness Scale, 2nd Edition (SRS-2), or any combination thereof.

The dosing frequency and dose amount per administration of a triptan may be selected to provide therapeutic effects for the treatment of one or more of the symptoms associated with ASD.

In some embodiments, the dosing frequency and dose amount per administration of a triptan are selected to provide therapeutic effects for the treatment of irritability symptoms associated with ASD.

The dosing frequency and dose amount per administration of a triptan may be selected to provide therapeutic effects for the treatment of aggression symptoms associated with ASD.

The dosing frequency and dose amount per administration of a triptan may selected to provide therapeutic effects for the treatment of the sociability symptoms associated with ASD.

In some embodiments, the methods of the present disclosure for treating the symptoms associated with autism comprise administering a therapeutically effective amount of zolmitriptan or a pharmaceutically acceptable salt thereof to a patient in need thereof. In some embodiments, the methods comprise administering about 1.0 mg to about 50 mg, including about 1.0 mg, about 1.25, about 1.5 mg, about 1.75 mg, about 2.0 mg, about 2.25 mg, about 2.5 mg, about 3.0 mg, about 3.5 mg, about 4.0 mg, about 4.5 mg, about 5.0 mg, about 5.5 mg, about 6.0 mg, about 6.5 mg, about 7.0 mg, about 7.5 mg, about 8.0 mg, about 8.5 mg, about 9.0 mg, about 9.5 mg, about 10.0 mg, about 10.5 mg, about 11.0 mg, about 11.5 mg, about 12.0 mg, about 12.5 mg, about 13.0 mg, about 13.5 mg, about 14.0 mg, about 14.5 mg, about 15.0 mg, about 15.5 mg, about 16.0 mg, about 16.5 mg, about 17.0 mg, about 17.5 mg, about 18.0 mg, about 18.5 mg, about 19.0 mg, about 19.5 mg, about 20.0 mg, about 20.5 mg, about 21.0 mg, about 21.5 mg, about 22.0 mg, about 22.5 mg, about 23.0 mg, about 23.5 mg, about 24.0 mg, about 24.5 mg, about 25.0 mg, about 25.5 mg, about 26.0 mg, about 26.5 mg, about 27.0 mg, about 27.5 mg, about 28.0 mg, about 28.5 mg, about 29.0 mg, about 29.5 mg, about 30.0 mg, about 30.5 mg, about 31 mg, about 31.5 mg, about 32 mg, about 32.5 mg, about 33 mg, about 33.5 mg, about 34 mg, about 34.5 mg, about 35 mg, about 35.5 mg, about 36 mg, about 36.5 mg, about 37 mg, about 37.5 mg, about 38 mg, about 38.5 mg, about 39 mg, about 39.5 mg, about 40 mg, about 40.5 mg, about 41 mg, about 41.5 mg, about 42 mg, about 42.5 mg, about 43 mg, about 43.5 mg, about 44 mg, about 44.5 mg, about 45.0 mg, about 45.5 mg, about 46 mg, about 46.5 mg, about 47 mg, about 47.5 mg, about 48 mg, about 48.5 mg, about 49.0 mg, about 49.5 mg, and about 50mg , including all values and ranges therebetween, of zolmitriptan or a pharmaceutically acceptable salt thereof to a patient in need thereof. In some embodiments, about 1.25 mg to about 35 mg of zolmitriptan or a pharmaceutically acceptable salt thereof is administered to a patient in need thereof. In some embodiments, about 1.7 mg to about 30 mg of zolmitriptan or a pharmaceutically acceptable salt thereof is administered to a patient in need thereof. In some embodiments, the methods of the disclosure comprise administering about 1.0 mg, about 1.25 mg, about 1.5 mg, about 1.75 mg, about 2.0 mg, about 2.25 mg, about 2.5 mg, about 3.0 mg, about 3.5 mg, about 4.0 mg, about 4.5 mg, about 5.0 mg, about 5.5 mg, about 6.0 mg, about 6.5 mg, about 7.0 mg, about 7.5 mg, about 8.0 mg, about 8.5 mg, about 9.0 mg, about 9.5 mg, about 10.0 mg, about 10.5 mg, about 11.0 mg, about 11.5 mg, about 12.0 mg, about 12.5 mg, about 13.0 mg, about 13.5 mg, about 14.0 mg, about 14.5 mg, about 15.0 mg, about 15.5 mg, about 16.0 mg, about 16.5 mg, about 17.0 mg, about 17.5 mg, about 18.0 mg, about 18.5 mg, about 19.0 mg, about 19.5 mg, about 20.0 mg, about 20.5 mg, about 21.0 mg, about 21.5 mg, about 22.0 mg, about 22.5 mg, about 23.0 mg, about 23.5 mg, about 24.0 mg, about 24.5 mg, about 25.0 mg, about 25.5 mg, about 26.0 mg, about 26.5 mg, about 27.0 mg, about 27.5 mg, about 28.0 mg, about 28.5 mg, about 29.0 mg, about 29.5 mg, about 30.0 mg, about 30.5 mg, about 31 mg, about 31.5 mg, about 32 mg, about 32.5 mg, about 33 mg, about 33.5 mg, about 34 mg, about 34.5 mg, about 35 mg, about 35.5 mg, about 36 mg, about 36.5 mg, about 37 mg, about 37.5 mg, about 38 mg, about 38.5 mg, about 39 mg, about 39.5 mg, about 40 mg, about 40.5 mg, about 41 mg, about 41.5 mg, about 42 mg, about 42.5 mg, about 43 mg, about 43.5 mg, about 44 mg, about 44.5 mg, about 45.0 mg, about 45.5 mg, about 46 mg, about 46.5 mg, about 47 mg, about 47.5 mg, about 48 mg, about 48.5 mg, about 49.0 mg, about 49.5 mg, or about 50mg of zolmitriptan or a pharmaceutically acceptable salt thereof to a patient in need thereof.

In some embodiments, about 2.0 mg to about 15 mg, including about 1.0 mg, about 1.5 mg, about 2.0 mg, about 2.5 mg, about 3.0 mg, about 3.5 mg, about 4.0 mg, about 4.5 mg, about 5.0 mg, about 5.5 mg, about 6.0 mg, about 6.5 mg, about 7.0 mg, about 7.5 mg, about 8.0 mg, about 8.5 mg, about 9.0 mg, about 9.5 mg, about 10.0 mg, about 10.5 mg, about 11 mg, about 11.5 mg, about 12 mg, about 12.5 mg, about 13 mg, about 13.5 mg, about 14 mg, about 14.5 mg, and about 15 mg, including all values and ranges therebetween of zolmitriptan or a pharmaceutically acceptable salt is administered to a patient three times per day. In some embodiments, about 2.0 mg to about 10 mg of zolmitriptan or a pharmaceutically acceptable salt thereof is administered to a patient three times per day.

In some embodiments, about 1.25 mg of zolmitriptan or a pharmaceutically acceptable salt thereof is administered to a patient in need thereof. In some embodiments, about 2.5 mg of zolmitriptan or a pharmaceutically acceptable salt thereof is administered to a patient in need thereof. In some embodiments, about 5 mg of zolmitriptan or a pharmaceutically acceptable salt thereof is administered to a patient in need thereof. In some embodiments, about 7.5 mg of zolmitriptan or a pharmaceutically acceptable salt thereof is administered to a patient in need thereof. In some embodiments, about 10 mg of zolmitriptan or a pharmaceutically acceptable salt thereof is administered to a patient in need thereof. In some embodiments, about 15 mg of zolmitriptan or a pharmaceutically acceptable salt thereof is administered to a patient in need thereof. In some embodiments, about 20 mg of zolmitriptan or a pharmaceutically acceptable salt thereof is administered to a patient in need thereof. In some embodiments, about 25 mg of zolmitriptan or a pharmaceutically acceptable salt thereof is administered to a patient in need thereof. In some embodiments, about 30 mg of zolmitriptan or a pharmaceutically acceptable salt thereof is administered to a patient in need thereof. In some embodiments, about 35 mg of zolmitriptan or a pharmaceutically acceptable salt thereof is administered to a patient in need thereof. In some embodiments, about 40 mg of zolmitriptan or a pharmaceutically acceptable salt thereof is administered to a patient in need thereof. In some embodiments, about 45 mg of zolmitriptan or a pharmaceutically acceptable salt thereof is administered to a patient in need thereof. In some embodiments, about 50 mg of zolmitriptan or a pharmaceutically acceptable salt thereof is administered to a patient in need thereof.

In some embodiments, zolmitriptan or a pharmaceutically acceptable salt thereof is administered once per day to a patient in need thereof. In some embodiments, zolmitriptan or a pharmaceutically acceptable salt thereof is administered twice per day to a patient in need thereof. In some embodiments, zolmitriptan or a pharmaceutically acceptable salt thereof is administered three times per day to a patient in need thereof.

In some embodiments, about 1.25 mg of zolmitriptan or a pharmaceutically acceptable salt thereof is administered once per day. In some embodiments, about 1.25 mg of zolmitriptan or a pharmaceutically acceptable salt thereof is administered twice per day. In some embodiments, about 1.25 mg of zolmitriptan or a pharmaceutically acceptable salt thereof is administered three times per day. In some embodiments, about 1.25 mg of zolmitriptan or a pharmaceutically acceptable salt thereof is administered four times per day.

In some embodiments, about 2.5 mg of zolmitriptan or a pharmaceutically acceptable salt thereof is administered once per day. In some embodiments, about 2.5 mg of zolmitriptan or a pharmaceutically acceptable salt thereof is administered twice per day. In some embodiments, about 2.5 mg of zolmitriptan or a pharmaceutically acceptable salt thereof is administered three times per day.

In some embodiments, about 5 mg of zolmitriptan or a pharmaceutically acceptable salt thereof is administered once per day. In some embodiments, about 5 mg of zolmitriptan or a pharmaceutically acceptable salt thereof is administered twice per day. In some embodiments, about 5 mg of zolmitriptan or a pharmaceutically acceptable salt thereof is administered three times per day.

In some embodiments, about 7.5 mg of zolmitriptan or a pharmaceutically acceptable salt thereof is administered once per day. In some embodiments, about 7.5 mg of zolmitriptan or a pharmaceutically acceptable salt thereof is administered twice per day. In some embodiments, about 7.5 mg of zolmitriptan or a pharmaceutically acceptable salt thereof is administered three times per day.

In some embodiments, about 10 mg of zolmitriptan or a pharmaceutically acceptable salt thereof is administered once per day. In some embodiments, about 10 mg of zolmitriptan or a pharmaceutically acceptable salt thereof is administered twice per day. In some embodiments, about 10 mg of zolmitriptan or a pharmaceutically acceptable salt thereof is administered three times per day.

In some embodiments, about 15 mg of zolmitriptan or a pharmaceutically acceptable salt thereof is administered once per day. In some embodiments, about 15 mg of zolmitriptan or a pharmaceutically acceptable salt thereof is administered twice per day. In some embodiments, about 15 mg of zolmitriptan or a pharmaceutically acceptable salt thereof is administered three times per day.

In some embodiments, about 20 mg of zolmitriptan or a pharmaceutically acceptable salt thereof is administered once per day. In some embodiments, about 20 mg of zolmitriptan or a pharmaceutically acceptable salt thereof is administered twice per day. In some embodiments, about 20 mg of zolmitriptan or a pharmaceutically acceptable salt thereof is administered three times per day.

In some embodiments, about 25 mg of zolmitriptan or a pharmaceutically acceptable salt thereof is administered once per day. In some embodiments, about 25 mg of zolmitriptan or a pharmaceutically acceptable salt thereof is administered twice per day. In some embodiments, about 25 mg of zolmitriptan or a pharmaceutically acceptable salt thereof is administered three times per day.

In some embodiments, about 30 mg of zolmitriptan or a pharmaceutically acceptable salt thereof is administered once per day. In some embodiments, about 30 mg of zolmitriptan or a pharmaceutically acceptable salt thereof is administered twice per day. In some embodiments, about 30 mg of zolmitriptan or a pharmaceutically acceptable salt thereof is administered three times per day.

In some embodiments, about 35 mg of zolmitriptan or a pharmaceutically acceptable salt thereof is administered once per day. In some embodiments, about 35 mg of zolmitriptan or a pharmaceutically acceptable salt thereof is administered twice per day. In some embodiments, about 35 mg of zolmitriptan or a pharmaceutically acceptable salt thereof is administered three times per day.

In some embodiments, about 40 mg of zolmitriptan or a pharmaceutically acceptable salt thereof is administered once per day. In some embodiments, about 40 mg of zolmitriptan or a pharmaceutically acceptable salt thereof is administered twice per day. In some embodiments, about 40 mg of zolmitriptan or a pharmaceutically acceptable salt thereof is administered three times per day.

In some embodiments, about 45 mg of zolmitriptan or a pharmaceutically acceptable salt thereof is administered once per day. In some embodiments, about 45 mg of zolmitriptan or a pharmaceutically acceptable salt thereof is administered twice per day. In some embodiments, about 45 mg of zolmitriptan or a pharmaceutically acceptable salt thereof is administered three times per day.

In some embodiments, about 50 mg of zolmitriptan or a pharmaceutically acceptable salt thereof is administered once per day. In some embodiments, about 50 mg of zolmitriptan or a pharmaceutically acceptable salt thereof is administered twice per day. In some embodiments, about 50 mg of zolmitriptan or a pharmaceutically acceptable salt thereof is administered three times per day.

In some embodiments, the methods of the present disclosure for treating the symptoms associated with autism further include a step of titrating the dose of zolmitriptan or a pharmaceutically acceptable salt thereof for at least about one week until a steady state is achieved in the patient. In some embodiments, the titration is conducted for about 2 weeks until a steady state is achieved in the patient. In some embodiments, the titration is conducted for about 7 days to about 30 days until a steady state is achieved in the patient. In some embodiments, the titration is conducted for about 12 days to about 20 days until a steady state is achieved in the patient.

In some embodiments, ascending doses of zolmitriptan or a pharmaceutically acceptable salt thereof are administered during the titration until a steady state is achieved in the patient. In some embodiments, ascending doses of zolmitriptan or a pharmaceutically acceptable salt thereof are administered during the titration until an effective amount of about 5 mg, about 7.5 mg, about 10.0 mg, about 12.5 mg, or about 15 mg is achieved in the patient.

In some embodiments, the titration is initiated with a dose of about 2.5 mg administered three times a day. In some embodiment, the titration is initiated with a dose of about 5 mg administered three times a day.

In some embodiments, the methods of the present disclosure for treating the symptoms associated with autism comprise initiating with a dose of about 2.5 mg of zolmitriptan or a pharmaceutically acceptable salt thereof administered three times a day and titrating to a dose of about 5 mg zolmitriptan or a pharmaceutically acceptable salt thereof administered three times a day. In some embodiments, the methods of the present disclosure for treating the symptoms associated with autism comprise initiating with a dose of about 5 mg of zolmitriptan or a pharmaceutically acceptable salt thereof three times a day and titrating to a dose of about 7.5 mg zolmitriptan or a pharmaceutically acceptable salt thereof administered three times a day. In some embodiments, the methods of the present disclosure for treating the symptoms associated with autism) comprise initiating with a dose of about 5 mg of zolmitriptan or a pharmaceutically acceptable salt thereof three times a day and titrating to a dose of about 10 mg zolmitriptan or a pharmaceutically acceptable salt thereof administered three times a day.

In some embodiments, the methods of the present disclosure provide CSF levels of zolmitriptan that correlate to one or more statistically significant therapeutic effects. In some embodiments, the therapeutically effective CSF levels of zolmitriptan provided by the methods of the present disclosure range from about 0.05 ng/mL to about 2 ng/ mL, including about 0.05 ng/mL, about 0.075 ng/mL, about 0.1 ng/mL, about 0.125 ng/mL, about 0.15 ng/mL, about 0.175 ng/mL, about 0.2 ng/mL, about 0.25 ng/mL, about 0.3 ng/mL, about 0.35 ng/mL, about 0.4 ng/mL, about 0.45 ng/mL, about 0.5 ng/mL, about 0.55 ng/mL, about 0.6 ng/mL, about 0.65 ng/mL, about 0.7 ng/mL, about 0.75 ng/mL, about 0.8 ng/mL, about 0.85 ng/mL, about 0.9 ng/mL, about 0.95 ng/mL, about 1 ng/mL, about 1.1 ng/mL, about 1.15 ng/mL, about 1.2 ng/mL, about 1.25 ng/mL, about 1.3 ng/mL, about 1.35 ng/mL, about 1.4 ng/mL, about 1.45 ng/mL, about 1.5 ng/mL, about 1.55 ng/mL, about 1.6 ng/mL, about 1.65 ng/mL, about 1.7 ng/mL, about 1.75 ng/mL, about 1.8 ng/mL, about 1.85 ng/mL, about 1.9 ng/mL, about 1.95 ng/mL, and about 2 ng/mL, including all values and ranges therebetween. In some embodiments, the therapeutically effective CSF levels of zolmitriptan provided by the methods of the present disclosure range from about 0.078 ng/mL to about 1.24 ng/ mL. In some embodiments, the therapeutically effective CSF levels of zolmitriptan provided by the methods of the present disclosure range from about 0.103 ng/mL to about 0.93 ng/ mL.

The triptan may be administered one or more times per day. In some embodiments, the triptan is administered once per day. The triptan may be administered twice per day. The triptan may be administered three times per day. The triptan may be administered more than three times per day.

In some embodiments, the present disclosure provides methods of treating the irritability associated with autism comprising administering a therapeutically effective amount of a triptan. The present disclosure may provide methods of treating the aggression associated with autism comprising administering an effective amount of a triptan or a pharmaceutically acceptable salt thereof. The present disclosure may provide methods of treating the lethargy associated with autism comprising administering an effective amount of a triptan or a pharmaceutically acceptable salt thereof. In some embodiments, the present disclosure provides methods of treating the sociability symptoms associated with autism comprising administering an effective amount of a triptan to improve socialization. In some embodiments, the present disclosure provides methods of reducing the social deficits associated with autism comprising administering an effective amount of a triptan or a pharmaceutically acceptable salt thereof.

After the treatment the patient may experience a substantial reduction in the symptoms associated with ASD (e.g. social symptoms, aggression or irritability) compared to prior to said treating. Non-limiting examples of ASD symptoms include irritability, difficulty with communication, difficulty with social interactions, obsessive interests, repetitive behaviors, inappropriate social interaction, poor eye contact, compulsive behavior, impulsivity, repetitive movements, self-harm, persistent repetition of words or actions learning disabilities, speech delays, intense interest in a limited number of things, problems paying attention, lack of awareness of others' emotions, depression, anxiety, changes in voice, sensitivity to sound, and tics. In some embodiments, the patient in need experiences a substantial decrease in irritability associated with ASD compared to prior to said treating. In some embodiments, the patient in need experiences a substantial improvement in sociability compared to prior to said treating. In some embodiments, the patient in need experiences a substantial decrease in aggression associated with ASD compared to prior to said treating.

The Vineland Adaptive Behavior Scales (VABS), third edition, is a standardized measure of adaptive behavior used to evaluate the personal and social skills of an individual from birth through adulthood. Individuals can be evaluated on the VABS scale by either teachers or caregivers. According to the VABS, an individual is assigned a VABS adaptive behavior composite score, which measures an individual's functioning compare to others of his or her age. An individual is also assigned domain scores in communication, daily living skills, socialization, and motor skills to assess an individual's adaptive behavior strengths and weaknesses. An individual receives a score from 20 to 140 on each of the domain scores and the VABS adaptive behavior composite score. A score from 20 to 70 represents low adaptive level. A score from 71 to 85 represents moderately low adaptive level. A score from 86 to 114 represents moderately adequate adaptive level. A score from 115 to 129 represents moderately high adaptive level. A score from 130 to 140 represents high adaptive level.

After said treatment the patient may experience a reduction of symptoms associated with ASD that is characterized by at least a one point increase in the VABS adaptive behavior composite score compared to prior to the treatment. The increase in the VABS adaptive behavior score may be about 1 point, about 2 points, about 3 points, about 4 points, about 5 points, about 6 points, about 7 points, about 8 points, about 9 points, about 10 points, about 11 points, about 12 points, about 13 points, about 14 points, about 15 points, about 16 points, about 17 points, about 18 points, about 19 points, about 20 points, about 21 points, about 22 points, about 23 points, about 24 points, about 25 points, about 26 points, about 27 points, about 28 points, about 29 points, about 30 points, about 31 points, about 32 points, about 33 points, about 34 points, about 35 points, about 36 points, about 37 points, about 38 points, about 39 points, about 40 points, about 41 points, about 42 points, about 43 points, about 44 points, about 45 points, about 46 points, about 47 points, about 48 points, about 49 points, about 50 points, about 51 points, about 52 points, about 53 points, about 54 points, about 55 points, about 56 points, about 57 points, about 58 points, about 59 points, about 60 points, about 61 points, about 62 points, about 63 points, about 64 points, about 65 points, about 66 points, about 67 points, about 68 points, about 69 points, or about 70 points compared to prior to said treating.

The increase in VABS adaptive behavior composite score may be at least about 1 %, at least about 5 %, at least about 10 %, at least about 15 %, at least about 20 %, at least about 25 %, at least about 30 %, at least about 35 %, at least about 40 %, at least about 45 %, at least about 50 %, at least about 55 %, or at least about 60 % compared to prior to said treating.

After said treatment the patient may experience an increase in sociability that is characterized by an increase in the VABS adaptive behavior socialization domain score compared to prior to the treatment. The increase in the VABS adaptive behavior socialization domain score may be about 1 point, about 2 points, about 3 points, about 4 points, about 5 points, about 6 points, about 7 points, about 8 points, about 9 points, about 10 points, about 11 points, about 12 points, about 13 points, about 14 points, about 15 points, about 16 points, about 17 points, about 18 points, about 19 points, about 20 points, about 21 points, about 22 points, about 23 points, about 24 points, about 25 points, about 26 points, about 27 points, about 28 points, about 29 points, about 30 points, about 31 points, about 32 points, about 33 points, about 34 points, about 35 points, about 36 points, about 37 points, about 38 points, about 39 points, about 40 points, about 41 points, about 42 points, about 43 points, about 44 points, about 45 points, about 46 points, about 47 points, about 48 points, about 49 points, about 50 points, about 51 points, about 52 points, about 53 points, about 54 points, about 55 points, about 56 points, about 57 points, about 58 points, about 59 points, about 60 points, about 61 points, about 62 points, about 63 points, about 64 points, about 65 points, about 66 points, about 67 points, about 68 points, about 69 points, or about 70 points compared to prior to said treating.

The increase in VABS adaptive behavior socialization domain score may be at least about 1 %, at least about 5 %, at least about 10 %, at least about 15 %, at least about 20 %, at least about 25 %, at least about 30 %, at least about 35 %, at least about 40 %, at least about 45 %, at least about 50 %, at least about 55 %, or at least about 60 %, compared to prior to said treating. The patient may experiences an at least 10 % improvement on the socialization domain score of VABS after treatment. The patient may experience an at least 35 % improvement on the socialization domain score of VABS after treatment.

After said treatment the patient may experience an improvement in communication that is characterized by an increase in the VABS adaptive behavior communication domain score compared to prior to the treatment. The increase in the VABS adaptive behavior communication domain may be about 1 point, about 2 points, about 3 points, about 4 points, about 5 points, about 6 points, about 7 points, about 8 points, about 9 points, about 10 points, about 11 points, about 12 points, about 13 points, about 14 points, about 15 points, about 16 points, about 17 points, about 18 points, about 19 points, about 20 points, about 21 points, about 22 points, about 23 points, about 24 points, about 25 points, about 26 points, about 27 points, about 28 points, about 29 points, about 30 points, about 31 points, about 32 points, about 33 points, about 34 points, about 35 points, about 36 points, about 37 points, about 38 points, about 39 points, about 40 points, about 41 points, about 42 points, about 43 points, about 44 points, about 45 points, about 46 points, about 47 points, about 48 points, about 49 points, about 50 points, about 51 points, about 52 points, about 53 points, about 54 points, about 55 points, about 56 points, about 57 points, about 58 points, about 59 points, about 60 points, about 61 points, about 62 points, about 63 points, about 64 points, about 65 points, about 66 points, about 67 points, about 68 points, about 69 points, or about 70 points, compared to prior to the treatment.

The increase in VABS adaptive behavior communication domain score may be at least about 1 %, at least about 5 %, at least about 10 %, at least about 15 %, at least about 20 %, at least about 25 %, at least about 30 %, at least about 35 %, at least about 40 %, at least about 45 %, at least about 50 %, at least about 55 %, or at least about 60 % compared to prior to said treating.

The Autism Diagnostic Observation Schedule, Second Edition (ADOS-2) is an instrument that allows accurate assessment and diagnosis of ASD across age, developmental level, and language skills. The ADOS-2 is a clinician-administered observational assessment that comprises two behavioral domains: social affect (SA) and restricted and repetitive behaviors (RRB). An individual is administered one of the five ADOS-2 modules, which is selected on the basis of the individual's expressive language level and chronological age. The toddler module is for children between 12 and 30 months of age who do not consistently use phrase speech. Module 1 is for children 31 months and older who do not consistently use phrase speech. Module 2 is for children of any age who use phrase speech but are not verbally fluent. Module 3 is for verbally fluent children and young adolescents. Module 4 is for verbally fluent older adolescents and adults. An individual is assigned an ADOS-2 Composite Total score ranging from 1 to 10. Individuals with ASD are characterized by an ADOS-2 Composite Total score between 6 and 10.

The patient may experience a reduction of symptoms associated with ASD associated with an at least one point decrease in the ADOS-2 Composite Total score compared to prior to said treating. The patient may experience an improvement in the symptoms of ASD that is characterized by a decrease of at least 1 point, at least 2 points, at least 3 points, at least 4 points, or at least 5 points on the ADOS-2 Composite Total score compared to prior to said treating.

The patient may experience an improvement in the symptoms of ASD that is characterized by an at least about 5 %, or at least about 10 %, or at least about 15 %, or at least about 20 %, or at least about 25 %, or at least about 30 %, or at least about 35 %, or at least about 40 %, or at least about 45 %, or at least about 50 %, or at least about 55 %, or at least about 60 %, or at least about 65 %, or at least about 70 %, or at least about 75 %, or at least about 80 %, or at least about 85 %, or at least about 90 % improvement in the ADOS-2 composite score compared to prior to said treating.

The patient may experience a reduction of symptoms associated with ASD that is characterized by an at least one point decrease in the ADOS-2 module 4 social affect score compared to prior to said treatment. In some embodiments, the decrease in the ADOS-2 module 4 social affect score is correlated with an improvement in sociability. The improvement in sociability may be characterized by an at least 1 point, or at least 2 point, or at least 3 point, or at least 4 point, or at least 5 point, or at least 6 point decrease in the social affect score on the ADOS-2 module 4 compared to prior to said treating.

The improvement in sociability may be characterized by an at least 10 %, or at least 15 %, or at least 20 %, or at least 25 %, or at least 30 %, or at least 35 %, or at least 40 %, or at least 45 %, or at least 50 %, or at least 55 %, decrease in the social affect scale on ADOS-2 module 4 compared to prior to said treating. The patient may experience an improvement in sociability that is characterized by an at least 10 % decrease in the social reciprocity score on the Autism Diagnostic Observation Schedule module 4 compared to prior to said treating.

The clinical global impression-severity (CGI-S) scale is utilized by clinicians to rate the severity of an ASD patient's symptoms. An individual is assigned a score of 1 to 7. A score of 1 represents a normal patient. A score of 7 represents the score of a patient with ASD.

After said treatment the patient may experience a reduction of symptoms associated with ASD that is characterized by at least a one point decrease in the CGI-S score compared to prior to said treating. The patient may experience an at least 1 point, or at least 2 points, or at least 3 points, or at least 4 points, or at least 5 points decrease in the CGI-S scale compared to prior to said treating.

The patient may experience an at least 5 %, or at least 10 %, or at least 15 %, or at least 20 %, or at least 25 %, or at least 30 %, or at least 35 %, or at least 40 %, or at least 45 %, or at least 50 %, or at least 55 %, or at least 60 %, or at least 65 %, or at least 70 %, or at least 75 %, or at least 80 %, or at least 85 %, or at least 90 % reduction in the CGI-S scale compared to prior to said treating.

The clinical global impression-severity (CGI-C) scale is utilized by clinicians to evaluate a change in an ASD patient's symptoms. An individual is assigned a score from 1 (very much improved) to 7 (very much worse).

After said treatment the patient may experience a reduction of symptoms associated with ASD that is characterized by at least a one point decrease in the CGI-C scale. The patient may experience an improvement in irritability that is characterized by a CGI-C score of $\leq 1$, $\leq 2$, $\leq 3$, or $\leq 4$ after said treatment. The patient's improvement in irritability is characterized by a Clinical Global Impression-Change (CGI-C) score of $\leq 3$ after said treatment. The patient may experience an improvement in sociability that is characterized by a CGI-C score of $\leq 1$, $\leq 2$, $\leq 3$, or $\leq 4$ after said treatment. The patient may experience an improvement in sociability that is characterized by a CGI-C score of $\leq 3$ after said treatment.

The Autism Behavior Inventory (ABI) social deficit subscale is a measure used for assessing changes in the core and associated symptoms of ASD. An individual is administered the ABI-full (93 items) or ABI-short version (36) scales. An individual is scored from 0 points to 6 points on each item of the scales, where 0 is the absence of symptoms and 6 is maximum symptoms. An individual that receives a score of 0 does not exhibit symptoms. An individual that is assigned a score of 6 experiences severe ASD symptoms.

The patient may experience an improvement in symptoms that is characterized by a decrease in ABI score of at least one point. The patient may experience an improvement in symptoms that is characterized by a decrease in ABI score of at least 1 point, or at least 2 points, or at least 3 points, or at least 4 points, compared to prior to said treating.

The patient may experience an improvement in symptoms that is characterized by an at least 5 %, or at least 10 %, or at least 15 %, or at least 20 %, or at least 25 %, or at least 30 %, or at least 35 %, or at least 40 %, or at least 45 %, or at least 50 %, or at least 55 %, or at least 60 %, or at least 65 %, or at least 70 %, or at least 75 %, or at least 80 %, or at least 85 %, or at least 90 %, or at least 95 % reduction in ABI score compared to prior to said treating.

The childhood autism rating scale (CARS) is used to identify children two years and older with ASD. The CARS consists of 14 domains assessing behaviors associated with ASD, with a 15th domain rating general impressions of autism. Each domain is scored on a scale ranging from one to four; higher scores are associated with a higher level of impairment. Total scores can range from a low of 15 to a high of 60; scores below 30 indicate that the individual is in the non-autistic range, scores between 30 and 36.5 indicate mild to moderate autism, and scores from 37 to 60 indicate severe autism.

After said treatment the patient may experience a reduction of symptoms associated with ASD that is characterized by a decrease in the CARS total score of about 1 point, about 2 points, about 3 points, about 4 points, about 5 points, about 6 points, about 7 points, about 8 points, about 9 points, about 10 points, about 11 points, about 12 points, about 13 points, about 14 points, about 15 points, about 16 points, about 17 points, about 18 points, about 19 points, about 20 points, about 21 points, about 22 points, about 23 points, about 24 points, about 25 points, about 26 points, about 27 points, about 28 points, about 29 points, or about 30 points, compared to prior to said treating.

After said treatment the patient may experience a reduction of symptoms associated with ASD that is characterized by a decrease in the CARS total score of at least 5 %, or at least 10 %, or at least 15 %, or at least 20 %, or at least 25 %, or at least 30 %, or at least 35 %, or at least 40 %, or at least 45 %, or at least 50 %, or at least 55 %, or at least 60 %, or at least 65 %, or at least 70 %, or at least 75 %, or at least 80 %, or at least 85 %, or at least 90 %, or at least 95 %, compared to prior to said treating.

The social responsiveness scale, 2nd Edition (SRS-2) is utilized to obtain an efficient quantitative measure of the various dimensions of interpersonal behavior, communication, and repetitive/stereotypic behavior associated with ASD. According to the SRS-2, an individual is assigned a proxy version total t-score, based on the SRS-2. An individual is assigned a proxy version t-score which reflects the sum of responses to 65 social responsiveness scale questions, which serves as an index of severity of social skills across the autism spectrum. An individual that receives a proxy version t-score

of greater than 76 receives a severe clinical diagnosis of ASD. If an individual is assigned a proxy version t-score of between 66 and 75, which is associated with moderate deficiencies in reciprocal social behavior that are clinically significant and lead to a substantial interference in everyday social interactions. If the individual is assigned a proxy version t-score between 60 to 65, the individual exhibits mild to moderate deficits in social interaction. If a patient exhibits a proxy version t-score of 59 or below, the patient exhibits normal social interactions.

Prior to administering a therapeutically effective amount of a triptan to a patient in need thereof, the patient in need thereof may exhibit a proxy version total t-score of ≥ 66.

The patient may experience an improvement in symptoms that is characterized by a decrease in proxy version total t-score of at least one point, or at least two points, or at least three points, or at least four points, or at least five points, or at least six points, or at least seven points, or at least eight points, or at least nine points, or at least ten points, or at least eleven points, or at least twelve points, or at least thirteen points, or at least fourteen points, or at least fifteen points, or at least sixteen points, or at least seventeen points, or at least eighteen points, or at least twenty points, or at least twenty one points, or at least twenty two points, or at least twenty three points, or at least twenty four points, or at least twenty five points, compared to prior to said treating.

The patient may experience an improvement in symptoms that is characterized by a decrease in proxy version total t-score of the SRS-2 of at least 5 %, or at least 10 %, or at least 15 %, or at least 20 %, or at least 25 %, or at least 30 %, or at least 35 %, or at least 40 %, or at least 45 %, or at least 50 %, or at least 55 %, or at least 60 %, or at least 65 %, or at least 70 %, or at least 75 %, or at least 80 %, or at least 85 %, or at least 90 %, or at least 95 %, compared to prior to said treating.

The social responsiveness scale for adults (SRS-A) is a tool to evaluate social responsiveness in adulthood. The SRS-A contains 65 items which are scored from 0 to 3. If an individual receives a score of 0, the individual does not have the symptom. If an individual receives a score of 3, the individual has a severe symptom. An SRS-A total score of 67 indicates that an individual has ASD. The maximum SRS-A total score is 195.

After said treatment the patient may experience a reduction of symptoms associated with ASD that is characterized by at least a one point decrease in the SRS-A score compared to prior to the treatment. The patient may experience an improvement in sociability that is characterized by an at least 1 point decrease in the SRS-A score. The patient's SRS-A score may decrease by about 1 point, or about 5 points, or about 10 points, or about 15 points, or about 20 points, or about 25 points, or about 30 points, or about 35 points after said treatment.

After said treatment the patient may exhibit a reduction in symptoms associated with ASD that is characterized by a decrease in the SRS-A score of about 5 %, about 10 %, about 15 %, about 20 %, about 25 %, about 30 %, about 35 %, about 40 %, about 45 %, about 50 %, about 55 %, about 60 %, about 65 %, about 70 %, compared to prior to said treatment. After said treatment the patient may exhibit a reduction in symptoms associated with ASD that is characterized by an at least 10 % decrease in the SRS-A score compared to prior to said treatment.

An improvement in sociability may be associated with an a decrease in the SRS-A score of about 10 %, about 15 %, about 20 %, about 25 %, about 30 %, about 35 %, about 40 %, or about 50 %, compared to prior to said treatment.

The aberrant behavior checklist (ABC) is a behavior rating scale that is utilized to rate individuals in five subscales: (1) irritability, agitation, and crying, (2) lethargy, social withdrawal, (3) stereotypic behavior, (4) hyperactivity and noncompliance, and (5) inappropriate speech. Individuals can be evaluated on the ABC by any adult that knows the individual well. The ABC contains a 58-item questionnaire that is utilized to assess the five-subscales. Each item on the 58-item questionnaire is rated from 0 to 3. A score of 0 means an absence in symptom. A score of 3 means an individual experiences the symptom with high severity. Items within each subscale are added to obtain a subscale score. Possible subscale scores on the ABC range from 0 to 48 with higher subscores indicating behavioral impairment.

After said treatment the patient may experience a reduction of symptoms associated with ASD that is characterized by at least a one point decrease in the ABC irritability agitation and crying (ABC-I) subscore. After said treatment the patient experiences a reduction in irritability that is characterized by a decrease in the ABC-I communication domain score compared to prior to the treatment.

The patient may exhibit an ABC-I score of ≥ 18 prior to treatment.

After said treatment the patient may experience a reduction in irritability that is characterized by an about 2 point, about 4 point, about 6 point, about 8 point, about 10 point, about 15 point, about 20 point, about 25 point, or about 30 point decrease in the ABC-I domain score compared to prior to said treatment. The decrease in the ABC-I domain score may be about 5 %, about 10 %, about 15 %, about 20 %, about 25 %, about 30 %, about 35 %, about 40 %, about 45 %, about 50 %, about 55 %, about 60 %, about 65 %, about 70 %, about 75 %, about 80 %, about 85 %, about 90 %, or about 95 % compared to prior to said treatment.

After said treatment the patient may experience a reduction of symptoms associated with ASD that is characterized by at least a one point decrease in the ABC-Lethargy and Social Withdrawal (ABC-LSW) subscore compared to prior to said treatment. After said treatment the patient may experience a reduction in lethargy and social withdrawal that is characterized by a decrease in the ABC-LSW communication domain score compared to prior to the treatment.

The decrease in the ABC-LSW communication domain score may be about 2 points, about 4 points, about 6 points, about 8 points, about 10 points, about 15 points, about 20 points, about 25 points, or about 30 points, compared to prior to said treatment.

The decrease in the ABC-LSW communication domain score may be about 5 %, about 10 %, about 15 %, about 20 %, about 25 %, about 30 %, about 35 %, about 40 %, about 45 %, about 50 %, about 55 %, about 60 %, about 65 %, about 70 %, about 75 %, about 80 %, about 85 %, about 90 %, or about 95 %, compared to prior to said treatment.

The social communication questionnaire is a tool used by physicians to screen patients with ASD. An individual's caregiver rates the verbal individual on a score of 0 to 39 or the non-verbal individual on a scale of 0 to 33. An individual that has ASD is characterized by a social communication questionnaire that is above 15.

After said treatment the patient may experience a reduction of symptoms associated with ASD that is characterized by an at least 10 %, at least 15 %, at least 20 %, at least 25 %, at least 30 %, at least 35 %, at least 40 %, at least 45 %, at least 50 %, at least 55 %, at least 60 %, at least 65 %, at least 70 %, at least 75 %, at least 80 %, at least 85 %, at least 90 %, or at least 95 % decrease in the social communication questionnaire scale compared to prior to said treatment.

The pervasive developmental disorder behavior inventory (PDDBI) is utilized to evaluate the efficacy of the treatment of ASD. The PDDBI assesses both problem behaviors and appropriate social, language, and learning/memory skills. The PDDBI is divided into two behavioral dimensions: (a) approach-withdrawal problems, assessing maladaptive behaviors and (b) receptive/expressive social communication abilities, assessing social communication competence. The approach-withdrawal problem dimension is further divided into behavioral domains, including sensory/perceptual approach behaviors, ritualisms/resistance to chain (RITUAL), social pragmatic problems (SOCPP), semantic/pragmatic problems (SEMPP), arousal regulation problems (AROUSE), specific fears (FEARS), and aggressiveness (AGG) domains. The receptive/expressive social communication abilities, assessing social communication competence (REXSCA) is further divided into behavioral domains, including the social approach behaviors (SOCAPP), expressive language (EXPRESS), and learning memory and receptive language (LMRL) domains. An individual receives a T score for each domain and a composite score, which is the sum of the scores in each domain. An individual with ASD is assigned a T score of greater than 50.

The pervasive developmental disorder behavior inventory (PDDBI) may be utilized to characterize the reduction in symptoms associated with ASD provided by the methods of the disclosure. A patient may exhibit a T score of greater than 50 prior to said treatment. After said treatment the patient may experience a reduction of symptoms associated with ASD that is characterized by a decrease in the PDDBI by about 5 %, or about 10 %, or about 15 %, or about 20 %, or about 25 %, or about 30 %, or about 35 %, or about 40 %, or about 45 %, or about 50 %, or about 55 %, or about 60 %, or about 65 %, or about 70 %, or about 75 %, or about 80 %, or about 85 %, or about 90 %, or about 95 % compared to prior to said treating.

The ATEC is a scale utilized to evaluate the efficacy of ASD treatments. The ATEC is a one-page form designed to be completed by parents, teachers, or caretakers. The ATEC consists of four subtests: speech/language communication, sociability, sensory/cognitive awareness, and health/physical/behavior. An individual is assigned a speech/language communication subtest rating from 0 to 28. An individual is assigned a sociability subtest rating from 0 to 40. An individual is assigned a sensory/cognitive awareness subtest rating from 0 to 36. An individual is assigned a health/physical/behavior subtest rating of 0 to 75. The individual subsets are summed, and the individual can receive a maximum score of 180 points After said treatment the patient may experience a reduction of symptoms associated with ASD that is characterized by a decrease in the mean ATEC score compared to prior to the treatment. After said treatment the patient may experience an improvement in speech/ language communication that is characterized by a decrease in the speech/language communication subtest rating compared to prior to said treating. After said treatment the patient may experience an improvement in sociability that is characterized by a decrease in the sociability subtest rating compared to prior to said treating. After said treatment the patient may experience an improvement in sensory/cognitive awareness that is characterized by a decrease in sensory/cognitive awareness subtest rating compared to prior to said treating. After said treatment the patient may experience an improvement in health/physical/behavior that is characterized by a decrease in health/physical/behavior rating, compared to prior to said treating.

The patient may exhibit a decrease on the sociability subsection of the ATEC of at least 1 point, or about 2 points, or about 3 points, or about 4 points, or about 5 points, or about 6 points, or about 7 points, or about 8 points, or about 9 points, or about 10 points, or about 11 points, or about 12 points, or about 13 points, or about 14 points, or about 15 points, or about 16 points, or about 17 points, or about 18 points, or about 19 points, or about 20 points compared to prior to said treating. The patient may exhibit a decrease on the sociability subsection of the ATEC of at least 1 point compared to prior to said treating.

The patient may exhibit a decrease on the sociability subsection of the ATEC of at least 5 %, or at least 10 %, or at least 15 %, or at least 20 %, or at least 25 %, or at least 30 %, or at least 35 %, or at least 35 %, or at least 40 %, or at least 50 % compared to prior to said treating. The patient may exhibit a decrease on the sociability subsection of the ATEC of at least 10 % compared to prior to said treating.

Objective/performance-based assessments are utilized to measure the reduction in the symptoms associated with ASD after administering a therapeutically effective amount of a triptan to a patient in need thereof. In some embodiments, the objective/performance-based assessments are selected from the group consisting of eye gaze tracking of social stimuli (eye tracking), parent engagement rating inventory (JERI), and Noldus Ethovision Analysis. In some embodiments, eye gaze tracking of social stimuli is utilized to characterize the reduction in symptoms associated with ASD provided by the methods of the disclosure. Patients with ASD exhibit significantly shorter look durations to the eyes (eye contact) as compared to their peers without ASD. Patients with ASD have more difficulty locating and processing pertinent social information the more rapidly stimuli are presented.

The PCIT provides training methods designed to help adults improve their parenting and language skills and to help children learn how to better control emotions. The parent child interaction task (PCIT) is utilized to improve the relationship between the caregiver and the patient with ASD. The PCIT may be helpful for reducing child behavior issues and increasing communication and interaction skills within the family. Children who participate in CPT may develop greater self-esteem, experience less anger and frustration, see an improvement in social, organizational, and play skills, feel safer and calmer, and communicate more effectively.

The Joint Engagement Ranking Inventory (JERI) is a tool which is utilized to measure targets for early invention for developmental disorders and delays, such as ASD. The JERI is an 18-item inventory developed to measure relevant intervention targets, such as unengagement, object engagement, joint engagement, stereotyped, restricted and repetitive behavior, attention to the caregiver, initiation of communication, expressive language level and use, scaffolding, following-in, caregiver affect, fluency and connectedness, and shared routines and rituals. An individual evaluated on the JERI scale receives a score from 1 to 7 on each item in the JERI inventory. A score of 7 is assigned to an individual with the most joint engagements. A score of 1 on an item is assigned to an individual that has no episodes of joint engagement. A lower JERI scale is correlated with ASD.

A reduction in the symptoms may be ASD is associated with lower scores on the JERI compared to prior to treatment by the methods of the disclosure. After said treatment the patient may experience a reduction of symptoms associated with ASD that is characterized by a decrease in the JERI score compared to prior to the treatment. The JERI scores may be reduced by about 10 %, about 20 %, about 30 %, about 40 %, about 50 %, about 60 %, or about 70 % compared to prior to the treatment.

The Ohio State University Autism Rating Scale (OARS-5) measures persistent impairment in social interactions, restrictive interests/activities and repetitions in behavior, and level of support from social, academic, and community (Hollway, J.A., Arnold, L.E., & Aman, M.G. (2017, September). OSU Autism Rating Scale - DSM-5 (OARS-5).). The OARS-5 was developed to provide three types of summary scores: (A) a Autism Symptom Count, based on clinical interview (OARS-5 Total Score); (b) a Weighted Mean Severity score based on severity of autism symptoms, derived from the clinical interview; (c) and an Impairment Index ranging from 0 to 9, based on the level of supports needed due to severity. The OARS-5 Total Score is equal to OARS-5 Social Deficits Subscale Score + OARS-5 Restricted Patterns of Interest Subscale Score.

The OARS-5 includes the following subscales:

- Section A: Persistent impairment in social interactions across multiple settings (OARS-5 Social Deficits Subscale Score);

- Section B: Restricted interests/activities and repetitive patterns of behavior (OARS-5 Restricted Patterns of Interest Subscale Score) and

- Section C: Level of support for Section A (social interactions/communication) and B (restricted and repetitive behaviors).

After said treatment the patient may experience a reduction of symptoms associated with ASD that is characterized by a decrease in the OARS-5 Total Score compared to prior to the treatment. After said treatment the patient may experience a reduction of symptoms associated with ASD that is characterized by a decrease of about 1 point, about 2 points, about 3 points, or about 4 points, about 5 points, about 6 points, about 7 points, about 8 points, about 9 points or about 10 points in the OARS-5 Total Score compared to prior to the treatment.

After said treatment the patient may experience a reduction of symptoms associated with ASD that is characterized by a decrease in the OARS-5 Social Deficits Subscale Score compared to prior to the treatment. After said treatment the patient may experience a reduction of symptoms associated with ASD that is characterized by a decrease of about 1 point, about 2 points, about 3 points, or about 4 points, about 5 points, about 6 points, about 7 points, about 8 points or about 9 points in the OARS-5 Social Deficits Subscale Score compared to prior to the treatment.

After said treatment the patient may experience a reduction of symptoms associated with ASD that is characterized by a decrease in the Ohio State University Autism Clinical Global Impression (OSU Autism CGI) total score compared to prior to the treatment. OSU Autism CGI-S total score is equal to OSU Autism CGI-Severity Scale (OSU Autism CGI-S) Score + OSU Autism CGI-Improvement Scale (OSU Autism CGI-I) Score.

After said treatment the patient may experience a reduction of symptoms associated with ASD that is characterized by a decrease of about 1 point, about 2 points, about 3 points, or about 4 points, about 5 points, about 6 points, or about 7 points in the OSU Autism CGI total score compared to prior to the treatment.

After said treatment the patient may experience a reduction of symptoms associated with ASD that is characterized by a decrease in the Ohio State University Autism Clinical Global Impression-Improvement Scale (OSU Autism CGI-I) score compared to prior to the treatment. After said treatment the patient may experience a reduction of symptoms associated with ASD that is characterized by a decrease of about 1 point, about 2 points, about 3 points, or about 4 points, about 5 points, about 6 points, or about 7 points in the OSU Autism CGI-I compared to prior to the treatment.

After said treatment the patient may experience a reduction of symptoms associated with ASD that is characterized by a decrease in the Ohio State University Autism Clinical Global Impression-Severity Scale (OSU Autism CGI-S) score compared to prior to the treatment. After said treatment the patient may experience a reduction of symptoms associated with ASD that is characterized by a decrease of about 1 point, about 2 points, about 3 points, or about 4 points, about 5 points, about 6 points, or about 7 points in the OSU Autism CGI-S compared to prior to the treatment.

*Patient Populations*

The disclosure provides methods of treating the symptoms associated with autism spectrum disorder (ASD) comprising administering a therapeutically effective amount of a triptan to a patient in need thereof.

The patient in need of a treatment for ASD may be a patient diagnosed with ASD according to the DSM-5 diagnostic criteria.

Prior to treatment according to the methods of the disclosure, the ASD patient in need thereof may have a full scale IQ score on the Weschsler Abbreviated Scale of Intelligence (WASI®)-II of $\geq$ 70.

The present disclosure provides methods of treating the symptoms associated with ASD that are refractory to treatment with existing ASD treatments. The symptoms associated with ASD may be refractory to treatment with aripiprazole. The symptoms associated with ASD may be refractory to treatment with risperidone.

The treated ASD patient may exhibit atypical sensory processing. Sensory processing refers to the ability to register, process, and organize sensory information and to execute appropriate responses to environmental demands, which manifest as hypersensitivities or hyposensitivities to stimuli. The patient in need with atypical sensory processing may have aversions to the color, taste, smell, and/ or texture of foods or medicines. Atypical sensory processing may be manifested in the patient in need as noncompliance with taking medications. The treated ASD patient may refuse to swallow medications. Said medications may be liquid formulations or pills.

In some embodiments, the treated ASD patient is an infant. In some embodiments, the treated ASD patient is a child. In some embodiments, the treated ASD patient is an adolescent. In some embodiments, the treated ASD patient is an adult. In some embodiments, the treated ASD patient is a geriatric patient

**EXAMPLES**

General protocols

In mouse pharmacokinetic experiments, the plasma Cmax for zolmitriptan occurred at the first time point measured (i.e., Tmax @ 15 minutes). The Tmax for IP injection of naratriptan, rizatriptan, frovatriptan and eletriptan also occurred 15 minutes (i.e., the first time point measured in those studies).

**Example 1.**

The effect of zolmitriptan on aggressive behavior was investigated in a mouse model.

The rodent Resident-Intruder assay (RI) has been used to monitor aggressive behaviors related to the behavioral patterns exhibited in establishing and defending territory (Miczek et al., 1984). Correspondingly, the RI assay has been used preclinically to study the effect of drugs on rodent aggression (Miczek et al., 2001). The RI assay typically relies on the interpretation and scoring of offensive (resident animal) behavior patterns and may also include analysis of defensive (intruder animal) behavior patterns as well.

Aggressive behaviors were evaluated using an unfamiliar, naive, age-matched "intruder" C57BL/6 mouse that was introduced into the home cage of a "resident" male BALB/c mouse of at least 8 weeks of age for a 5 min interaction. The resident mice were isolated for 1 week prior to testing aggressive behavior, while the intruders were group housed (4 per cage) throughout the study. All mice were given ad libitum food and water.

Following the 1-week isolation period, aggressive behaviors were recorded using a front-facing camera daily (Monday-Friday) for up to 2 weeks to attain a stable baseline prior to evaluating drug effects. The interaction was scored using Borris open source event logging software to score the number of total initiated attacks by the resident and the cumulative duration of those attacks. Single fight bouts lasting longer than 60 seconds were stopped and the intruder was subsequently removed from the resident cage. In addition, if the intruder showed aggressive behavior, the recordings were stopped and the intruder was removed from the resident cage.

The BALB/c-C57BL/6 RI combination was used to evaluate the effect of 3 and 10 mg/kg of zolmitriptan on aggression. The resident mice were treated with vehicle for two consecutive days and then balanced for a two arm (3 and 10mg/kg) cross over study. On the third day (n=10) mice were treated with 3mg/kg and n=10 mice received 10mg/kg of zolmitriptan. The consecutive fourth and fifth days were vehicle treatment followed by a final cross over treatment day on the sixth day. The pretreatment time was 15 minutes and drugs were delivered via intraperitoneal (i.p.) injection.

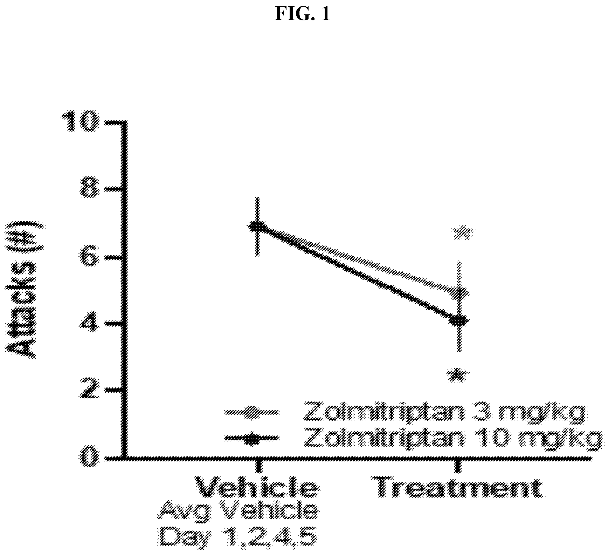

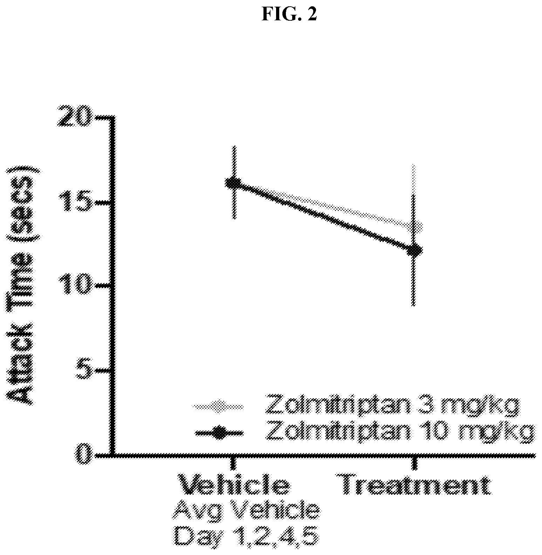

**Results:** As shown in **FIG. 1** and **FIG. 2,** the BALB/c mice showed a reduction in the number of attacks and attack time with zolmitriptan treatment. A significant dose dependent effect of reduction in attack behavior with zolmitriptan treatment after the completion of the cross-over study was found ($p<0.05$, Paired t-test). Additionally, zolmitriptan doses of 3 and 10 mg/kg did not effect normal locomotor activity in the treated mice.

## Example 2

A CD-1-C57BL/6 combination was used to evaluate the effect of zolmitriptan (10 mg/kg) on aggressive behavior in CD-1 mice using a rodent RI assay, which was carried out using the protocol described in Example 1. Risperidone (0.3 mg/kg) was used as a comparator compound. Attack time was measured over a 5 min period as a cross over design.

Zolmitriptan dosed at 3 and 10 mg/kg (i.p.) produced Cmax values of 5.40 and 34.42 ng/ml in CSF in adult male CD-1 mice, respectively. N-desmethyl zolmitriptan, ("NDMZ", which is the primary metabolite of zolmitriptan found in humans) was below the lower limit of quantification in male CD-1 mice dosed at 3 and 10 mg/kg (i.p.).

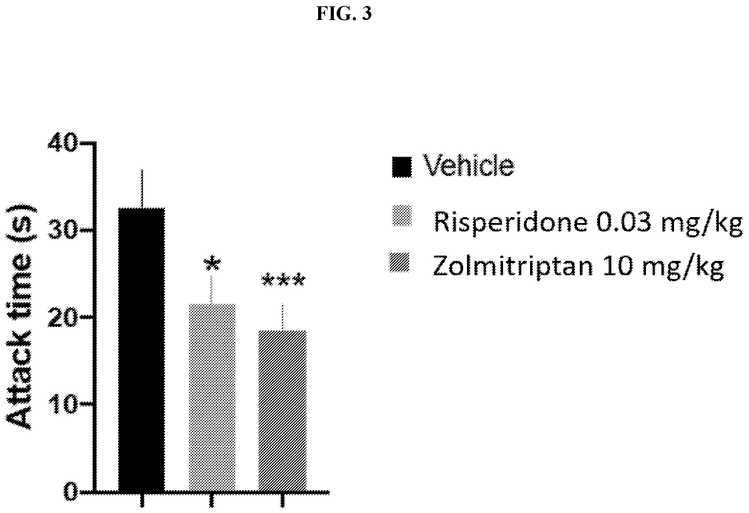

**Results:** As shown in **FIG. 3** (and tabulated below), CD-1 mice administered zolmitriptan at 10 mg/kg showed a greater reduction in attack time (s) compared to risperidone (0.03 mg/kg) and vehicle control.

| Treatment | N | Attack Time (s) | SEM | Latency to Attack (s) | SEM |
|---|---|---|---|---|---|
| Vehicle | 25 | 32.65 | 4.219 | 4.705 | 1.524 |
| Risperidone 0.03 mg/kg | 25 | 21.58* | 3.123 | 12.98 | 4.18 |
| Zolmitriptan 10mg/kg | 25 | 18.46*** | 3.028 | 13.54 | 5.520 |

## Example 3.

The effect of zolmitriptan in a mouse model of valproic acid-induced autism spectrum disorder was investigated (Nicolini C et al., 2018 Exp. Neurol., 2018, 217-227; Bey A. L., and Jiang J. H, Current Protocols in Pharmacology, 01 Sep 2014, 66:5.66.1-26). Specifically, sociability was evaluated in male c57/Bl6 mice that were previously exposed to valproic acid (VPA) via i.p. delivery of 500mg/kg to their pregnant mothers at embryonic Day 13.

In this study, zolmitriptan was dissolved in 10% (2-Hydroxypropyl)-β-cyclodextrin in saline and administered intraperitoneally (i.p.) 15 minutes before mice were placed in the test chamber. Behavioral recordings were measured using an automated video tracking system (Noldus EthoVision v14) and analyzed and graphed using GraphPad Prism software v8. Sociability was assessed during a 10 min period in the 3-chamber Social Interaction Assay. The time spent (sec) in the social interaction zone (SIZ) in front of the juvenile novel C57/BL6 vs empty interaction zone (EIZ) were used to calculate the sociability scores for individual mice. Sociability index was calculated using SIZ/EIZ ratio. Social interaction preference was calculated using (SIZ)/(SIZ+EIZ) *100. Simultaneous recording up to 4 arenas were conducted for the social interaction assay. Paired and unpaired T tests were used as statistical tests.

Zolmitriptan dosed at 10 mg/kg (i.p.) produced a Cmax value of 17.83 ng/ml (15 min after administration) in the CSF of adult male c57/Bl6 mice (i.e., Tg2576 background strain and strain used for VPA-treatment). NDMZ was below the lower limit of quantification in the CSF of male c57/Bl6 mice dosed at 10 mg/kg (i.p.).

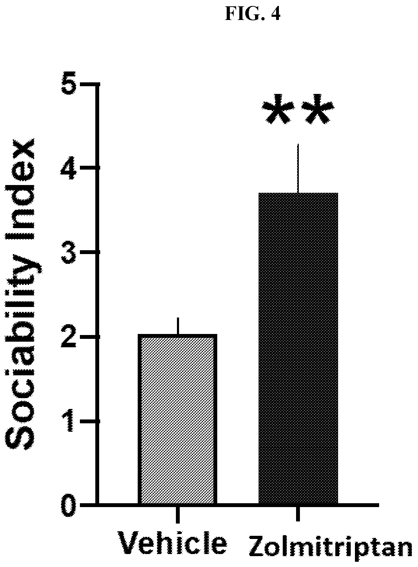

As shown in **FIG. 4** zolmitriptan dosed at 10 mg/kg (i.p.) increased the sociability index in adult male c57/Bl6 mice previously exposed to valproic acid (VPA). The improvement in sociability of the zolmitriptan treated mice was statistically significant compared to the vehicle treated group.

## Example 4

An investigation of the safety, tolerability and pharmacokinetic response of oral zolmitriptan following multiple ascending doses in adult healthy volunteer subjects was undertaken.

Zolmitriptan 2.5 mg per tablet or placebo tablet was administered 3 times per day (TID) for an up-titration period, treatment period of 7 days, and a down-titration period. The dose regimen is depicted in Table 1.

**Table 1**

| Cohort | Up-titration | Treatment (7 days) | Down-titration |
|---|---|---|---|
| 1 | 2.5 mg TID (2 days) | 5.0 mg TID | 2.5 mg TID (2 days) |
| 2 | 2.5 mg TID (2 days), 5.0 mg TID (2 days) | 10.0 mg TID | 5.0 mg TID (2 days), 2.5 mg TID (2 days) |
| 3 | 5.0 mg TID (2 days), 10.0 mg TID (2 days) | 20.0 mg TID | 10.0 mg TID (2 days), 5.0 mg TID (2 days) |
| 4 | 5.0 mg TID (1 day), 10.0 mg TID (2 days), 20.0 mg TID (2 days) | 30.0 mg TID | 20.0 mg TID (2 days), 10.0 mg TID (2 days), 5.0 mg TID (1 day) |
| 5 | 5.0 mg TID (1 day), 10.0 mg TID (2 days) 20.0 mg TID (2 days) | 40.0 mg TID | 20.0 mg TID (2 days), 10.0 mg TID (2 days), 5.0 mg TID (1 day) |

A lumbar puncture was completed on each subject to determine the concentration of zolmitriptan in the CSF. CSF samples were collected on day 5 of treatment for Cohort 1 and day 7 of treatment for Cohorts 2 and 3, and day 8 of treatment for cohort 4 and 5. The CSF collection was 2 hours after the morning dose (+/- 30 minutes). CSF concentration was measured using a validated bioanalytical method.

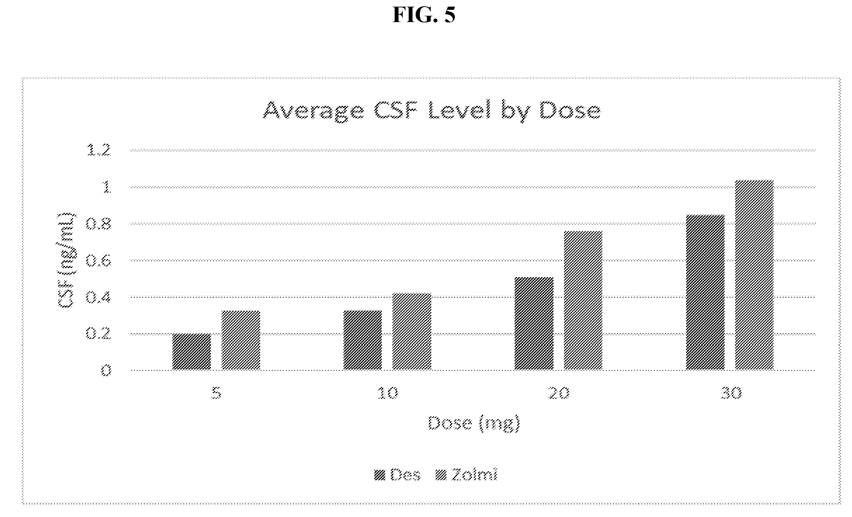

**Results:** As shown in **FIG. 5,** CSF levels measured in human exist in a ratio of zolmitriptan: NDMZ = 1.0: 0.75. Overall, Zolmitriptan was safe and well tolerated in all Cohorts.

**Example 5.**

Human PK studies that correlate zolmitriptan oral dose administration to CSF concentrations (Example 4, *i.e.,* mg zolmitriptan administered to CSF Cmax achieved), effective CSF concentrations determined from the valproate mouse model of ASD (Example 3) and species-specific binding assay data (human and mouse) were used to estimate effective human doses for treating the symptoms of ASD with zolmitriptan.

Specifically, 5-HT1b receptor occupancy and efficacy models measured potency of zolmitriptan and NMDZ at the 5-HT1b receptor and the ratio of zolmitriptan: NDMZ in human CSF. The 5-HT1b receptor efficacy model was used to predict the level of 5-HT1b activation (i.e., efficacy) by both zolmitriptan and NDMZ based on the concentration of zolmitriptan in the CSF (Table 5, column 2). From this target zolmitriptan dose ranges required to achieve an effective CSF exposures in humans were calculated (Table 5, column 3). Persons skilled in the art can apply similar methods to determine the effective doses for treating ASD symptoms with the triptans described herein.

**35S-GTPγS Binding Assay**

In this study *in vitro* binding assays were conducted to measure the affinity of zolmitriptan, its active metabolite NDMZ, eletriptan, naratriptan, and rizatriptan to rat and human 5-HT1B receptors.

Zolmitriptan, Eletriptan, Naratriptan, and Rizatriptan were tested in a SPA-based 35S-GTPγS binding assay in cells expressing human 5-HT1b (h5-HT1b) receptors or rat recombinant 5-HT1b (r5-HT1b) receptors at ten concentrations in duplicate. The EC50 vales are shown in Table 2.

**Table 2.** Functional Assay

| Triptan | h5-HT1b EC50 (nM) | r5-HT1b EC50 (nM) |
|---|---|---|
| Zolmitriptan | 4 | 33 |
| Eletriptan | 8.8 | 120 |
| Naratriptan | 1.2 | 7.4 |
| Rizatriptan | 38 | 210 |

All triptans exhibited lower apparent affinity (i.e., EC50) at rat 5-HT1b receptors than human 5-HT1b receptors. In the case of zolmitriptan, the difference between species is 8.3-fold.

Mouse and rat orthologs of 5-HT1b are 98% identical. They differ by only two amino acids, both of which are conservative changes and are removed from the agonist binding pocket (i.e., mouse/rat E152D in intracellular loop 1 and mouse/rat M192V in extracellular loop 2).

Based on this data, the CSF levels required for zolmitriptan to provide a therapeutic effect in humans is about 8.3 fold lower than the CSF levels required to provide similar activity in mice.

Assuming that CSF values mirror the free concentration of drugs in the brain, the estimated effective Cmax CSF values can be expressed as:

CD-1 (mouse aggression model, Example 2); 3 mg/Kg (i.p.); CSF Cmax = 18.8 nM or EC36 (36% activity in GTPgS)

c57/Bl6 (ASD mouse model, Example 3); 10mg/Kg (i.p.); CSF Cmax = 62 nM EC65 (65% activity in GTPgS)

## Radioligand Binding Competition Assay

Zolmitriptan, NDMZ (the active metabolite of Zolmitriptan in humans), Eletriptan, Naratriptan, Rizatriptan, and Frovatriptan and were evaluated for affinity for the human 5-HT1B receptor using a radioligand binding competition assay with 3H-5-CT and recombinant human 5-HT1B receptor at ten concentrations in duplicate. The affinity of the test compounds to the h5-HT1b receptor is shown in Table 3. The Ki values were derived from IC50 values using Cheng-Prusoff equation.

**Table 3. Binding Assay**

| Test compound | Ki (nM) |
|---|---|
| Zolmitriptan | 3.34 |
| NDMZ | 1.18 |
| Eletriptan | 4.48 |
| Naratriptan | 0.95 |
| Rizatriptan | 23.4 |
| Frovatriptan | 3.36 |

## 5-HT1b Receptor Occupancy and Efficacy Models

Using classical receptor theory, the receptor occupancy when considering two ligands (i.e., entities) can be predicted using the following relationship:

Total % occupancy = (% occupancy by A) + {[100% - (% occupancy by A)] x (% occupancy by B)} when considering a fixed concentration of both A and B.

Given that the ratio of zolmitriptan: NDMZ in humans is 1.0: 0.75 in human CSF (**FIG. 5**) and that NDMZ is 2.83-fold more potent than zolmitriptan, a model of 5-HT1b occupancy was constructed using the Total % occupancy formula above as the basis. The model was converted to predict the level of 5-HT1b activation (i.e., efficacy) by both zolmitriptan and NDMZ based on the concentration of zolmitriptan in the CSF by substituting Occupancy for Efficacy as predicted by the Clark equation {Efficacy = [L]/([L] + Ki); [L] = ligand concentration} for both zolmitriptan and NDMZ:

$$\text{Total \% Efficacy} = \{[\text{Zolmitriptan}]/([\text{Zolmitriptan}] + \text{Ki,Z})\} +$$

$$(100 - \{[\text{Zolmitriptan}]/([\text{Zolmitriptan}] + \text{Ki,Z})\} \text{ x } \{[\text{NDMZ}]/([\text{NDMZ}] + \text{Ki,N})\}$$

Using the measured EC50 value for Zolmitriptan in the GTPgS assay NMDZ's 2.83-fold greater affinity for the human 5-HT1b receptor, and the measured relationship of Zolmitriptan: NDMZ = 1.0 : 0.75, Total % Efficacy was calculated for a range of zolmitriptan concentrations and the resulting predicted Total % Efficacy was fitted with a logistic equation:

Total % Efficacy =100/(1+10^((LogEC50-[Zolmitriptan]))) and Log EC50 is calculated to be -8.963.The concentration of zolmitriptan in human CSF predicted to induce 50% efficacy (i.e., EC50) of 5-HT1b by zolmitriptan and NDMZ is 1.09 nM or 0.31 ng/ml.

The concentration of zolmitriptan required to achieve 50% Total Efficacy is 3.7-fold that of the zolmitriptan EC50 because of the combined effect of the potent metabolite.

**Zolmitriptan Human Dose Range** The dose required to achieve the predicted effective CSF concentration range in humans occurs across a range of 5-HT1b activity shown in Table 4.

**Table 4.**

| Activity | Zolmitriptan [CSF] | Zolmitriptan human dose (mg) |
|---|---|---|
| EC20 - EC80 | 0.078 - 1.24 ng/ml | 1.25 - 35 |
| EC25 - EC75 | 0.103 - 0.93 ng/ml | 1.7 - 30 |
| EC30 - EC70 | 0.135 - 0.713 ng/ml | 2.25 - 20 |
| EC35 - EC65 | 0.167 - 0.577 ng/ml | 3 - 15 |
| EC40 - EC60 | 0.207 - 0.465 ng/ml | 5 - 12.5 |

## Dose Predictions for other Triptans

The CSF: plasma ratio for zolmitriptan exposure in human is 3.0% (vs. 0.76% in mouse). There is no evidence that other triptans exhibit this species-specific bias.

Dose predictions can be made on the basis of the respective 5-HT1b EC50s of other triptans and the mouse CSF Cmax by relating them to the zolmitriptan values (Tables 3 & 5). While, other triptans are not thought to produce a potent, active metabolite like zolmitriptan (i.e. NMDZ), the contribution of any potential active metabolites for other triptans can be derived using 5-HT1b Receptor Occupancy and Efficacy Models similar to those disclosed herein for Zolmitriptan.

**Table 5.**

| Test compound | c57/Bl6 mouse CSF Cmax (10 mg/Kg, i.p.) | c57/Bl6 mouse CSF: plasma AUC (10 mg/Kg, i.p.) |
|---|---|---|
| Zolmitriptan | 17.83 ng/mL | 0.76% |
| Eletriptan | 13.46 ng/mL | 0.77% |
| Naratriptan | 79.56 ng/mL | 3.12% |
| Rizatriptan | 90.20 ng/mL | 4.66% |
| Frovatriptan | 12.52 ng/mL | 0.15% |

## Claims

1. A therapeutically effective amount of zolmitriptan or a pharmaceutically acceptable salt thereof for use in decreasing irritability or improving sociability associated with autism spectrum disorder (ASD).

2. The therapeutically effective amount of zolmitriptan or a pharmaceutically acceptable salt thereof for use according to claim 1, wherein prior to said treatment the patient is diagnosed with ASD using the DSM-5 diagnostic criteria.

3. The therapeutically effective amount of zolmitriptan or a pharmaceutically acceptable salt thereof for use according to any one of claims 1-2, wherein prior to said treatment the patient's Aberrant Behavior Checklist Irritability Subscale (ABC-I) score is $\geq$ 18, the patient's Social Responsiveness Scale, 2nd Edition (SRS-2), proxy version total t-score is $\geq$ 66, the patient's full scale IQ score on the Wechsler Abbreviated Scale of Intelligence (WASI®)-II is $\geq$ 70, and/or the patient has severe or moderate irritability and/or aggression.

4. The therapeutically effective amount of zolmitriptan or a pharmaceutically acceptable salt thereof for use according to any one of claims 1-3, wherein prior to the treatment the patient has moderate lethargy and/or social deficits.

5. The therapeutically effective amount of zolmitriptan or a pharmaceutically acceptable salt thereof for use according to any one of claims 1-4, wherein patient's symptoms are refractory to treatment with aripiprazole and risperidone.

6. The therapeutically effective amount of zolmitriptan or a pharmaceutically acceptable salt thereof for use according to any one of claims 1-5, wherein the patient is an adolescent, a child, or an adult.

7. The therapeutically effective amount of zolmitriptan or a pharmaceutically acceptable salt thereof for use according to any one of claims 1-6, wherein after said treating the patient experiences an improvement in sociability that is **characterized by** an at least 10% decrease in the sociability subsection of the ATEC, an at least 10% decrease in the social reciprocity score on the Autism Diagnostic Observation Schedule module 4, an at least 10% decrease in SRS-A score, and/or a Clinical Global Impression-Change (CGI-C) score of ≤ 3compared to prior to the treatment.

8. The therapeutically effective amount of zolmitriptan or a pharmaceutically acceptable salt thereof for use according to any one of claims 1-7, wherein after said treating the patient experiences an improvement in irritability associated with ASD that is **characterized by** an at least 10% decrease in ABC-I score, an at least 35% decrease in ABC-I score, a Clinical Global Impression-Change (CGI-C) score of ≤ 3, and/or an at least 10% improvement in the socialization domain score of the Vineland 3 Adaptive Behavior Scale compared to prior to the treatment.

9. The therapeutically effective amount of zolmitriptan or a pharmaceutically acceptable salt thereof for use according to any one of claims 1-8, wherein after said treating the patient experiences an improvement in symptoms of ASD that is **characterized by** a **characterized by** an at least 10% improvement in the communication domain score of the Vineland 3 Adaptive Behavior Scale, an at least one point improvement in Autism Diagnostic Observation Schedule, Second Edition (ADOS-2) Composite Total score, an at least one point decrease in OARS-5 Total Score, an at least one point decrease in OARS-5 Social Deficits Subscale Score, and/or an at least one point decrease in OSU Autism CGI total score compared to prior to the treatment.

10. The therapeutically effective amount of a triptan for use according to any one of claims 1-9, wherein after said treating the patient experiences an improvement in symptoms of ASD that is **characterized by** an at least one point decrease in OSU Autism CGI-I score or CGI-S score compared to prior to the treatment.

11. The therapeutically effective amount of zolmitriptan or a pharmaceutically acceptable salt thereof for use according to any one of claims 1-10, wherein about 1 mg to about 50 mg of zolmitriptan or a pharmaceutically acceptable salt thereof is administered to the patient.

12. The therapeutically effective amount of zolmitriptan or a pharmaceutically acceptable salt thereof for use according to any one of claims 1-11, wherein zolmitriptan or a pharmaceutically acceptable salt thereof is administered once per day.

13. The therapeutically effective amount of zolmitriptan or a pharmaceutically acceptable salt thereof for use according to any one of claims 1-12, wherein the administration provides a CSF level of about 0.05 ng/mL to about 2 ng/mL of zolmitriptan.

## Revendications

1. Quantité thérapeutiquement efficace de zolmitriptan ou d'un sel pharmaceutiquement acceptable de celui-ci, destinée à être utilisée pour diminuer l'irritabilité ou améliorer la sociabilité associées aux troubles du spectre de l'autisme (TSA).

2. Quantité thérapeutiquement efficace de zolmitriptan ou d'un sel pharmaceutiquement acceptable de celui-ci selon la revendication 1, le patient, avant ledit traitement, ayant reçu un diagnostic de TSA selon les critères diagnostiques du DSM-5.

3. Quantité thérapeutiquement efficace de zolmitriptan ou d'un sel pharmaceutiquement acceptable de celui-ci selon l'une quelconque des revendications 1 à 2, le patient, avant ledit traitement, présentant un score ABC-I (sous-échelle d'irritabilité selon la liste de contrôle des comportements aberrants) ≥ 18, un score t total version proxy de l'échelle de réactivité sociale, 2e édition (SRS-2) ≥ 66, un score de QI complet sur l'échelle d'intelligence abrégée de Wechsler (WASI®)-II ≥ 70, et/ou une irritabilité et/ou une agressivité sévères ou modérées.

4. Quantité thérapeutiquement efficace de zolmitriptan ou d'un sel pharmaceutiquement acceptable de celui-ci selon l'une quelconque des revendications 1 à 3, le patient, avant le traitement, présentant une léthargie et/ou des déficits sociaux modérés.

5. Quantité thérapeutiquement efficace de zolmitriptan ou d'un sel pharmaceutiquement acceptable de celui-ci selon l'une quelconque des revendications 1 à 4, les symptômes du patient étant réfractaires au traitement par l'aripiprazole et la rispéridone.

6. Quantité thérapeutiquement efficace de zolmitriptan ou d'un sel pharmaceutiquement acceptable de celui-ci selon l'une quelconque des revendications 1 à 5, le patient étant un adolescent, un enfant ou un adulte.

7. Quantité thérapeutiquement efficace de zolmitriptan ou d'un sel pharmaceutiquement acceptable de celui-ci selon l'une quelconque des revendications 1 à 6, le patient, après ledit traitement, présentant une amélioration de la sociabilité qui est **caractérisée par** une diminution d'au moins 10 % de la sous-section de sociabilité de l'ATEC, une diminution d'au moins 10 % du score de réciprocité sociale sur le module 4 du programme d'observation diagnostique de l'autisme, une diminution d'au moins 10 % du score SRS-A et/ou un score de changement d'impression clinique globale (CGI-C) $\leq$ 3 par rapport aux valeurs obtenues avant le traitement.

8. Quantité thérapeutiquement efficace de zolmitriptan ou d'un sel pharmaceutiquement acceptable de celui-ci selon l'une quelconque des revendications 1 à 7, le patient, après ledit traitement, présentant une amélioration de l'irritabilité associée aux TSA, qui est **caractérisée par** une diminution d'au moins 10 % du score ABC-I, une diminution d'au moins 35 % du score ABC-I, un score de changement d'impression clinique globale (CGI-C) $\leq$ 3 et/ou une amélioration d'au moins 10 % du score du domaine de socialisation de l'échelle de comportement adaptatif de Vineland 3 par rapport aux valeurs obtenues avant le traitement.

9. Quantité thérapeutiquement efficace de zolmitriptan ou d'un sel pharmaceutiquement acceptable de celui-ci selon l'une quelconque des revendications 1 à 8, le patient, après ledit traitement, présentant une amélioration des symptômes de TSA, qui est **caractérisée par** une amélioration d'au moins 10 % du score du domaine de la communication de l'échelle de comportement adaptatif Vineland 3, une amélioration d'au moins un point du score total composite du programme d'observation diagnostique de l'autisme (Autism Diagnostic Observation Schedule, ADOS-2, deuxième édition), une diminution d'au moins un point du score total OARS-5, une diminution d'au moins un point du score de la sous-échelle des déficits sociaux OARS-5 et/ou une diminution d'au moins un point du score total CGI de l'autisme OSU par rapport aux valeurs obtenues avant le traitement.

10. Quantité thérapeutiquement efficace d'un triptan destinée à être utilisée selon l'une quelconque des revendications 1 à 9, le patient, après ledit traitement, présentant une amélioration des symptômes de TSA qui est **caractérisée par** une diminution d'au moins un point du score CGI-I ou CGI-S de l'autisme OSU par rapport aux valeurs obtenues avant le traitement.

11. Quantité thérapeutiquement efficace de zolmitriptan ou d'un sel pharmaceutiquement acceptable de celui-ci selon l'une quelconque des revendications 1 à 10, environ 1 mg à environ 50 mg de zolmitriptan ou d'un sel pharmaceutiquement acceptable de celui-ci étant administrés au patient.

12. Quantité thérapeutiquement efficace de zolmitriptan ou d'un sel pharmaceutiquement acceptable de celui-ci selon l'une quelconque des revendications 1 à 11, le zolmitriptan ou un sel pharmaceutiquement acceptable de celui-ci étant administré une fois par jour.

13. Quantité thérapeutiquement efficace de zolmitriptan ou d'un sel pharmaceutiquement acceptable de celui-ci selon l'une quelconque des revendications 1 à 12, l'administration entraînant un taux de zolmitriptan dans le LCR d'environ 0,05 ng/ml à environ 2 ng/ml.

**Patentansprüche**

1. Therapeutisch wirksame Menge von Zolmitriptan oder einem pharmazeutisch akzeptablen Salz davon zur Verwendung zur Verringerung der Reizbarkeit oder zur Verbesserung der Soziabilität im Zusammenhang mit der Autismus-Spektrum-Störung (ASS).

2. Therapeutisch wirksame Menge von Zolmitriptan oder einem pharmazeutisch akzeptablen Salz davon zur Verwendung nach Anspruch 1, wobei vor der Behandlung bei dem Patienten ASS unter Verwendung der DSM-5-Diagnosekriterien diagnostiziert wird.

3. Therapeutisch wirksame Menge von Zolmitriptan oder einem pharmazeutisch akzeptablen Salz davon zur Verwendung nach einem der Ansprüche 1-2, wobei vor der Behandlung der ABC-I-Score (Aberrant Behavior Checklist Irritability Subscale) des Patienten $\geq 18$ beträgt, der Gesamt-T-Score des Patienten auf der Social Responsiveness Scale, 2nd Edition (SRS-2), Proxy-Version, $\geq 66$ beträgt, der IQ-Score des Patienten auf der vollen Skala der Wechsler Abbreviated Scale of Intelligence (WASI®)-I $\geq 70$ beträgt und/oder der Patient eine schwere oder mäßige Reizbarkeit und/oder Aggressivität aufweist.

4.  Therapeutisch wirksame Menge von Zolmitriptan oder einem pharmazeutisch akzeptablen Salz davon zur Verwendung nach einem der Ansprüche 1-3, wobei der Patient vor der Behandlung mäßige Lethargie und/oder soziale Defizite aufweist.

5.  Therapeutisch wirksame Menge von Zolmitriptan oder einem pharmazeutisch akzeptablen Salz davon zur Verwendung nach einem der Ansprüche 1-4, wobei die Symptome des Patienten gegenüber einer Behandlung mit Aripiprazol und Risperidon refraktär sind.

6.  Therapeutisch wirksame Menge von Zolmitriptan oder einem pharmazeutisch akzeptablen Salz davon zur Verwendung nach einem der Ansprüche 1-5, wobei der Patient ein Jugendlicher, ein Kind oder ein Erwachsener ist.

7.  Therapeutisch wirksame Menge von Zolmitriptan oder einem pharmazeutisch akzeptablen Salz davon zur Verwendung nach einem der Ansprüche 1-6, wobei der Patient nach der Behandlung eine Verbesserung der Soziabilität erfährt, die durch eine Abnahme des Soziabilitätsunterabschnitts des ATEC um mindestens 10%, eine Abnahme des sozialen Reziprozitäts-Scores auf dem Autism Diagnostic Observation Schedule Modul 4 um mindestens 10%, eine Abnahme des SRS-A-Scores um mindestens 10% und/oder einen Score auf der Clinical Global Impression-Change (CGI-C) von $\leq 3$ im Vergleich zu vor der Behandlung gekennzeichnet ist.

8.  Therapeutisch wirksame Menge von Zolmitriptan oder einem pharmazeutisch akzeptablen Salz davon zur Verwendung nach einem der Ansprüche 1-7, wobei der Patient nach der Behandlung eine Verbesserung der Reizbarkeit in Verbindung mit ASS erfährt, die durch eine Abnahme des ABC-I-Scores um mindestens 10%, eine Abnahme des ABC-I-Scores um mindestens 35%, einen Score auf der Clinical Global Impression-Change (CGI-C) von $\leq 3$ und/oder eine Verbesserung des Scores der Sozialisationsdomäne auf der Vineland 3 Adaptive Behavior Scale um mindestens 10 % im Vergleich zu vor der Behandlung gekennzeichnet ist.

9.  Therapeutisch wirksame Menge von Zolmitriptan oder einem pharmazeutisch akzeptablen Salz davon zur Verwendung nach einem der Ansprüche 1-8, wobei der Patient nach der Behandlung eine Verbesserung der Symptome von ASS erfährt, die durch eine Verbesserung des Scores der Kommunikationsdomäne auf der Vineland 3 Adaptive Behavior Scale um mindestens 10%, eine Verbesserung des kombinierten Gesamt-Scores auf dem Autism Diagnostic Observation Schedule, Second Edition (ADOS-2) um mindestens einen Punkt, eine Abnahme des OARS-5 Gesamt-Scores um mindestens einen Punkt, eine Abnahme des Scores auf der OARS-5 Social Deficits Subscale um mindestens einen Punkt und/oder eine Abnahme des OSU Autism CGI Gesamt-Scores um mindestens einen Punkt im Vergleich zu vor der Behandlung gekennzeichnet ist.

10.  Therapeutisch wirksame Menge eines Triptans zur Verwendung nach einem der Ansprüche 1-9, wobei der Patient nach dieser Behandlung eine Verbesserung der Symptome von ASS erfährt, die durch eine Abnahme des OSU-Autismus-CGI-I-Scores oder des CGI-S-Scores um mindestens einen Punkt im Vergleich zu vor der Behandlung gekennzeichnet ist.

11.  Therapeutisch wirksame Menge von Zolmitriptan oder einem pharmazeutisch akzeptablen Salz davon zur Verwendung nach einem der Ansprüche 1-10, wobei dem Patienten etwa 1 mg bis etwa 50 mg Zolmitriptan oder eines pharmazeutisch akzeptablen Salzes davon verabreicht wird.

12.  Therapeutisch wirksame Menge von Zolmitriptan oder einem pharmazeutisch akzeptablen Salz davon zur Verwendung nach einem der Ansprüche 1-11, wobei Zolmitriptan oder ein pharmazeutisch akzeptables Salz davon einmal täglich verabreicht wird.

13.  Therapeutisch wirksame Menge von Zolmitriptan oder einem pharmazeutisch akzeptablen Salz davon zur Verwendung nach einem der Ansprüche 1-12, wobei die Verabreichung einen CSF-Spiegel von etwa 0,05 ng/ml bis etwa 2 ng/ml Zolmitriptan bereitstellt.

**FIG. 1**

**FIG. 2**

FIG. 3

EP 4 048 249 B1

FIG. 4

FIG. 5

Average CSF Level by Dose

■ Des  ■ Zolmi

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- British Association for Psychopharmacology. *Journal of psychopharmacology*, 2000, vol. 22 (2), 1-5
- **SCHAIN et al.** *J Pediatr.*, March 1961, vol. 58, 315-20
- **KRAVITZ et al.** *Physiology (Bethesda)*, June 2012, vol. 27 (3), 167-77
- *Pfaff. Trends Neurosci.*, July 2019, vol. 42 (7), 448-457
- **KANE et al.** *PLoS One.*, 2012, vol. 7 (11), e48975
- **CHALLIS et al.** *J Neurosci.*, 28 August 2013, vol. 33 (35), 13978-88, 13988a
- **LI et al.** *Nat Commun.*, 28 January 2016, vol. 7, 10503
- **WALSH et al.** *Nature*, 2018, vol. 560 (772), 589-594
- **CLEMENTS et al.** *JAMA Psychiatry*, 01 August 2018, vol. 75 (8), 797-808
- **GHANIZADEN AHMAD et al.** *Pediatric neurology*, 2015, vol. 52 (1), 77-81
- **GARCIA-ALLOZA et al.** *Neuropsychologia.*, 2005, vol. 43 (3), 442-9
- **VARNAS et al.** *Hum Brain Mapp.*, July 2004, vol. 22 (3), 246-60
- **VARNAS et al.** *Synapse.*, vol. 56, 21-8
- **SARI.** *Neurosci Biobehav Rev.*, October 2004, vol. 28 (6), 565-82
- **BOSCHER et al.** *Neuroscience*, 1994, vol. 58, 167-182
- **HOLLWAY, J.A.** ; **ARNOLD, L.E.** ; **AMAN, M.G.** *OSU Autism Rating Scale - DSM-5 (OARS-5).*, September 2017
- **NICOLINI C et al.** *2018 Exp. Neurol.*, 2018, 217-227
- **BEY A. L.** ; **JIANG J. H**. *Current Protocols in Pharmacology*, 01 September 2014, vol. 66 (5.66), 1-26